# EUROPEAN PATENT APPLICATION

(11) **EP 4 847 493 A1**
(43) Date of publication of application: **02.12.2026**
(21) Application number: 24926925.9
(22) Date of filing: 31.12.2024
(51) Int. Cl.: C07D 277/34, C07D 417/06, A61P 35/00, A61P 35/02, A61P 3/10, A61P 37/02, A61K 31/427, A61K 31/4439, A61K 31/4709

(54) **THIAZOLIDINEDIONE HDAC INHIBITOR, PREPARATION METHOD THEREFOR AND USE THEREOF**

(30) Priority: 28.02.2024 CN 202410218999
(71) Applicant: Nanjing Hongshun Pharmaceutical Technology Co., Ltd, Nanjing, Jiangsu 210000 (CN); China Pharmaceutical University, Nanjing, Jiangsu 210000 (CN)
(72) Inventor: ZHOU, Qingfa, Nanjing, Jiangsu 210000 (CN); YAO, Shengfa, Nanjing, Jiangsu 210000 (CN); ZHANG, Yufei, Nanjing, Jiangsu 210000 (CN); WANG, Enyuan, Nanjing, Jiangsu 210000 (CN); JIA, Shuolei, Nanjing, Jiangsu 210000 (CN); RUAN, Xiuqin, Nanjing, Jiangsu 210000 (CN)
(74) Representative: Bayramoglu et al.
(86) International application number: PCT/CN2024/144068
(87) International publication number: WO 2025/180067

(57) **Abstract**

The present invention relates to a thiazolidinedione-based histone deacetylase (HDAC) inhibitor, a method for preparing the same and use thereof, and belongs to the technical field of medicines. The thiazolidinedione-based HDAC inhibitor of the present invention has a novel structure and serves as an efficient HDAC inhibitor, with the beneficial effects of efficient and excellent HDAC inhibitory activity and excellent in vitro anti-tumor activity, providing a novel and effective compound entity for the use of an HDAC inhibitor in solid tumors or for the treatment of diseases related to uncontrolled HDAC activity.

## Description

### TECHNICAL FIELD

The present invention relates to a thiazolidinedione-based HDAC inhibitor, a method for preparing the same and use thereof, and belongs to the technical field of medicines.

### BACKGROUND TECHNOLOGY

Traditional therapies for malignant tumors mainly include surgery, chemotherapy and radiotherapy. Although the surgery is an effective method, it is not ideal for prolonging the life of a patient if other adjuvant therapies are not further carried out. The chemotherapy may lead to drug resistance and cause side effects, typically including bone marrow suppression, impaired intestinal function, etc. The radiotherapy is a localized therapy that can inhibit tumor growth, but may also affect and cause damage to surrounding tissues. Molecular targeted therapy has emerged as a new approach for treating cancer due to its high specificity and low toxicity. Epigenetics is the study of mechanisms that alter the individual development and pass such an alternation on to offsprings by modifying genetic materials such as DNA through methylation, acetylation and chromatin conformation alternation without affecting the DNA sequence. Epigenetic regulation is crucial in many biological processes, and is closely related to the onset and progression of various human diseases such as cancer. The human genome contains 20,000 to 25,000 genes, only 5% of which are encoded by DNA sequences. However, not every gene corresponds to a single protein, since after various processes such as transcription, a messenger ribonucleic acid (mRNA) leads to a transcriptome that is several timers larger than the number of genes. This includes further amplification that occurs through the post-translational modification (PTM) of amino acid side chains after protein synthesis. Most PTMs are inherently dynamic, enabling a single protein to exist in various functional states and to transition from one state to another state in a reversible manner. In particular, the ε-amino side chain of lysine can undergo various PTMs such as alkylation or acylation to produce a variety of modified proteins. For example, the alkylation increases the length of the side chain of lysine while maintaining its positive charge at physiological pH, and the acylation produces neutral or even negatively charged side chains, with a range from formylation to the binding of proteins such as ubiquitin. Among these PTMs, acetylation and methylation often occur at the lysine-rich N-terminal of histones, and play a central role in epigenetic regulation of chromatin structures and ligand recruitment in a gene transcription process. Therefore, enzymes involved in lysine acetylation, methylation, and deacetylation or demethylation have become important targets for the discovery of small molecule drugs. Lysine acetylation was first observed in histones in the early 1960s. It can significantly alter the biological properties of proteins, for example, altering protein binding and stability and reducing the catalytic activity of related enzymes, etc. Shortly thereafter, enzymes that catalyze acetylation and deacetylation reactions were discovered, and named histone acetyltransferases (HATs) and histone deacetylases (HDACs). Malignant tumors are currently one of the greatest threats to humanity, and people have been actively seeking ways to alleviating and curing these diseases across various fields. In terms of chemotherapy, numerous studies have demonstrated that inhibiting histone deacetylases shows good therapeutic effects for many cancers. Despite more than thirty years of studies, existing clinical candidate HDAC inhibitors still have problems such as poor pharmacokinetic properties, average efficacy and limited indications, and the combined advantages of HDAC targets have not been fully exploited. Moreover, the types of HDAC inhibitors on the market remain limited. Therefore, this target still has high research value and potential.

### CONTENT OF THE INVENTION

In view of the defects existing in the prior art, an object of the present invention is to provide a thiazolidinedione-based HDAC inhibitor, a method for preparing the same and use thereof, in order to identify high-activity HDAC inhibitors for the preparation of tumor inhibition medicaments, providing a promising prospect for the development of anti-tumor medicaments.

The present invention solves the technical problems by means of the following technical solutions: the present invention first provides a thiazolidinedione-based HDAC inhibitor, including a substituted thiazolidinedione compound of Formula V, or a stereisomer, hydrate or pharmaceutically acceptable salt thereof:

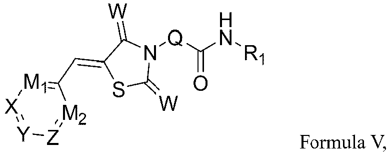

wherein in the formula, W is selected from the group consisting of an oxygen atom and a sulfur atom; X, Y, Z, M₁ and M₂ each independently represent a carbon or nitrogen atom, when X, Y, Z, M₁ or M₂ is a carbon atom, each is independently and optionally substituted by R₂, and R₂ can be hydrogen, alkyl, cyano, halogen, haloalkyl, hydroxy, mercapto, alkoxy, alkylthio, alkoxyalkyl, arylalkyl, aryl or Het;
Q is selected from the group consisting of a saturated or unsaturated straight or branched hydrocarbon group having a length of 1 to 9 carbon atoms, aryl and Het;
R₁ is selected from the group consisting of hydroxy, and 2-aminophenyl optionally substituted by one or more R₃;
R₃ can be hydrogen, alkyl, cyano, halogen, haloalkyl, hydroxy, mercapto, alkoxy, alkylthio, alkoxyalkyl, arylalkyl, aryl or Het;
the alkyl is a straight or branched saturated hydrocarbon group having 1 to 9 carbon atoms, or a cyclic saturated hydrocarbon group having 3 to 6 carbon atoms, or a cyclic saturated hydrocarbon group having 3 to 6 carbon atoms and linked to a straight or branched saturated hydrocarbon group having 1 to 6 carbon atoms;
the alkoxy is a straight or branched saturated hydrocarbon group having 1 to 9 carbon atoms, or a cyclic saturated hydrocarbon group having 3 to 9 carbon atoms, or a cyclic saturated hydrocarbon group having 3 to 6 carbon atoms and linked to a straight or branched saturated hydrocarbon group having 1 to 9 carbon atoms, wherein each carbon atom is optionally substituted by an oxygen atom;
the alkylamino is a straight or branched saturated hydrocarbon group having 1 to 9 carbon atoms, or a cyclic saturated hydrocarbon group having 3 to 6 carbon atoms, or a cyclic saturated hydrocarbon group having 3 to 6 carbon atoms and linked to a straight or branched saturated hydrocarbon group having 1 to 9 carbon atoms, wherein each carbon atom is optionally substituted by an NH atom;
the alkoxyalkyl represents linking the alkoxy defined as above to alkyl;
alkenyl or alkynyl is a straight or branched unsaturated hydrocarbon group having 1 to 9 carbon atoms and a double or triple bond;
the aryl is a carboatomic ring selected from the group consisting of phenyl, naphthyl, acenaphthenyl and tetrahydronaphthyl, each of which is optionally substituted by 1, 2 or 3 substituents, with each substituent independently selected from the group consisting of hydrogen, alkyl, cyano, halogen, haloalkyl, hydroxy, mercapto, alkoxy, alkylthio, alkoxyalkyl, arylalkyl, diarylalkyl, aryl and Het;
the arylalkyl or diarylalkyl represents linking the aryl defined as above to alkyl;
the Het is a monoheterocyclic ring selected from the group consisting of pyrrolyl, pyrazolyl, imidazolyl, furyl, thienyl, oxazolyl, isoxazolyl, thiazolyl, isothiazolyl, pyridinyl, pyrimidinyl, pyrazinyl and pyridazinyl, or is a biheterocyclic ring selected from the group consisting of quinolyl, quinoxalinyl, indolyl, benzoimidazolyl, benzoxazolyl, benzoisoxazolyl, benzothiazolyl, benzoisothiazolyl, benzofuryl, benzothienyl, 2,3-dihydrobenzo[1,4]dioxin and benzo[1,3]dioxol, or is selected from the group consisting of a monocyclic saturated hydrocarbon group having 3 to 6 carbon atoms, and a bicyclic saturated hydrocarbon group having 6 to 12 carbon atoms, wherein a carbon atom on a ring is independently and optionally substituted by 1 to 4 O, S, N or NH; and each monocyclic or bicyclic ring is optionally substituted by 1, 2 or 3 substituents, with each substituent independently selected from the group consisting of halogen, haloalkyl, hydroxy, alkyl and alkoxy.

The halogen is a substituent selected from the group consisting of fluorine, chlorine, bromine and iodine; and the haloalkyl is a straight or branched saturated hydrocarbon group having 1 to 9 carbon atoms, or a cyclic saturated hydrocarbon group having 3 to 6 carbon atoms, or a cyclic saturated hydrocarbon group having 3 to 6 carbon atoms and linked to a straight or branched saturated alkyl group having 1 to 6 carbon atoms, wherein one or more carbon atoms are substituted by one or more halogen atoms.

Preferably, a compound as below is further selected in the present invention as the thiazolidinedione-based HDAC inhibitor: in the formula, W is selected from the group consisting of an oxygen atom and a sulfur atom; X, Y, Z, M₁ and M₂ each independently represent a carbon or nitrogen atom, when X, Y, Z, M₁ or M₂ is a carbon atom, each is independently and optionally substituted by R₂, and R₂ is hydrogen, alkyl, cyano, halogen, haloalkyl, alkoxy, alkylthio, or alkoxyalkyl;
Q is selected from the group consisting of a saturated or unsaturated straight or branched hydrocarbon group having a length of 1 to 9 carbon atoms, and aryl;
R₁ is selected from the group consisting of hydroxy, and 2-aminophenyl optionally substituted by one or more R₃; and
R₃ is hydrogen, alkyl, cyano, halogen, haloalkyl, aryl or Het.

Preferably, a compound as below is further selected in the present invention as the thiazolidinedione-based HDAC inhibitor: in the Formula, W is selected from the group consisting of an oxygen atom and a sulfur atom; X, Y, Z, M₁ and M₂ each independently represent a carbon or nitrogen atom, when X, Y, Z, M₁ or M₂ is a carbon atom, each is independently and optionally substituted by R₂, and R₂ is hydrogen, alkyl, halogen or alkoxy;
Q is selected from the group consisting of a saturated or unsaturated straight or branched hydrocarbon group having a length of 3 to 9 carbon atoms, and aryl;
R₁ is selected from the group consisting of hydroxy, and 2-aminophenyl optionally substituted by one or more R₃; and
R₃ is hydrogen, alkyl, cyano, halogen, haloalkyl, aryl or Het.

Further, the hydrogen attached to the carbon in the formula can be replaced by deuterium, which is an isotope of hydrogen. For example, the alkyl can be replaced by deuterated alkyl, the alkoxy can be replaced by deuterated epoxy, a benzene ring can be replaced by a deuterated benzene ring, and an aromatic ring can be replaced by a deuterated aromatic ring.

A pharmaceutically acceptable salt refers to a salt obtained by converting a basic group in a parent compound into a salt form. The pharmaceutically acceptable salt includes, but is not limited to, inorganic or organic acid salts of basic groups such as amino (amine) groups. The pharmaceutically acceptable salt of the present invention can be synthesized from a parent compound by reacting the basic group in the parent compound with 1-4 equivalents of an acid in a solvent system. Suitable salts are listed in Remington's Pharmaceutical Sciences, 17th ed., Mack Publishing Company, Easton, Pa., 1985, p. 1418 and Journal of Pharmaceutical Science, 66, 2 (1977).

The basic group of the compound of the present invention may form a salt with an acid, and examples of the acid salt include: a salt formed with an inorganic acid, in particular a hydrohalic acid (such as hydrochloric acid, hydrobromic acid, or hydroiodic acid), nitric acid, sulfuric acid, phosphoric acid, carbonic acid, etc.; a salt formed with a lower alkylsulfonic acid, such as methanesulfonic acid or trifluoromethanesulfonic acid; a salt formed with an arylsulfonic acid, such as benzenesulfonic acid or p-toluenesulfonic acid; a salt formed with an organic acid, such as acetic acid, fumaric acid, tataric acid, oxalic acid, citric acid, maleic acid, malic acid or succinic acid; or a salt formed with an amino acid, such as aspartic acid or glutamic acid.

The compound of the present invention and the pharmaceutically acceptable salt thereof further include a solvate or hydrate form. In general, solvate or hydrate forms are equivalent to non-solvated or non-hydrated forms and are encompassed within the scope of the present invention. Some compounds in the present invention may exist in polycrystalline or amorphous forms. In short, all physical forms have equivalent uses and are encompassed within the scope of the present invention.

In addition, unless otherwise indicated, the structural formula of the compound in the thiazolidinedione-based HDAC inhibitor of the present invention includes all isomeric forms (such as enantiomers, diastereomers, and geometric isomers (or conformational isomers)), for example, R or S configurations containing asymmetric centers, (*Z*) or (*E*) isomers of double bonds, and (*Z*) or (*E*) conformational isomers. Therefore, the single stereochemical isomers of the compounds of the present invention or the mixtures of their enantiomers, diastereomers or geometric isomers (or conformers) are all within the scope of the present invention.

The term "alkyl" involved in the present application refers to a saturated straight or branched monovalent hydrocarbon group containing 1 to 20 carbon atoms, and the alkyl can be independently and optionally substituted by one or more substituents described in the present application. In some embodiments, the alkyl group contains 1 to 10 carbon atoms; in other embodiments, the alkyl group contains 1 to 8 carbon atoms; in further embodiments, the alkyl group contains 1 to 6 carbon atoms; and in other further embodiments, the alkyl group contains 1 to 4 carbon atoms. Further examples of the alkyl group include, but are not limited to, methyl (Me, -CH₃), ethyl (Et, -CH₂CH₃), n-propyl (n-Pr, -CH₂CH₂CH₃), isopropyl (i-Pr, -CH(CH₃)₂), n-butyl (n-Bu, -CH₂CH₂CH₂CH₃), iso-butyl (i-Bu, -CH₂CH(CH₃)₂), sec-butyl (s-Bu, - CH(CH₃)CH₂CH₃), tert-butyl (t-Bu, -C(CH₃)₃), etc. The term "alkyl" and its prefix "alk-" as used herein include both straight and branched saturated carbon chains.

The term "alkoxy" has an alkyl moiety that has the same definition as the aforementioned "alkyl", and it is formed by attaching an oxygen atom to the main carbon chain of the "alkyl" group.

The term "haloalkyl" or "haloalkoxy" represents the fact that an "alkyl" or "alkoxy" group may be substituted by one or more identical or different halogen atoms. Here, the alkyl and alkoxy groups have the same meanings as described above, and such examples include, but are not limited to, trifluoromethyl, trifluoromethoxy, etc.

The term "hydroxyalkyl" or "hydroxyalkoxy" represents the fact that an "alkyl" or "alkoxy" group may be substituted by one or more hydroxy groups. Here, the "alkyl" and "alkoxy" groups have the same meanings as described above, and such examples include, but are not limited to, hydroxymethyl, 1-hydroxyethyl, hydroxypropyl, 1,2-dihydroxypropyl, hydroxymethoxy, 1-hydroxyethoxy, etc.

The term "halogen" or "halogen atom" includes fluorine, chlorine, bromine and iodine.

The term "heterocyclyl" may be a carbon group or a heteroatom group. The "heterocyclyl" group also includes groups formed by combining a heterocyclic group with a saturated or partially unsaturated ring or heterocyclic ring. Examples of the heterocyclic ring include, but are not limited to, pyrrolidinyl, tetrahydrofuranyl, dihydrofuranyl, tetrahydrothienyl, tetrahydropyranyl, dihydropyranyl, tetrahydrothiapyranyl, piperidinyl, thioxanyl, azetidinyl, oxetanyl, thietanyl, piperidinyl, homopiperidinyl, glycidyl, azepanyl, oxepanyl, thiepany, N-morpholinyl, 2-morpholinyl, 3-morpholinyl, thiomorpholinyl, N-piperazinyl, 2-piperazinyl, 3-piperzainyl, homopiperazinyl, 4-methoxy-piperidin-1-yl, 1,2,3,6-tetrahydropyridin-1yl, oxazepinyl, diazepinyl, thiazepinyl, pyrrolin-1-yl, 2-pyrrolinyl, 3-pyrrolinyl, dihydroindolinyl, 2H-pyranyl, 4H-pyranyl, dioxanyl, 1,3-dioxolanyl, pyrazolinyl, dithianyl, dithiolanyl, dihydrothienyl, pyrazolidinylimidazolinyl, imidazolidinyl, 1,2,3,4-tetrahydroisoquinolinyl, 1,2,6-thiadiazinane, 1,1-dioxo-2-yl, quinolizinyl and N-pyridylurea. Moreover, the heterocyclyl group may be substituted or unsubstituted, with a substituent that may be, but is not limited to, oxo(=O), hydroxy, amino, halogen, cyano, heteroaryl, alkoxy, alkylamino, alkyl, alkenyl, alkynyl, heterocyclyl, mercapto, nitro, aryloxy, hydroxy-substituted alkoxy, hydroxy-substituted alkyl-C(=O)alkyl-C(=O), carboxylalkoxy, etc.

Based on the formula described above, the thiazolidinedione-based HDAC inhibitor of the present invention has one of the following structures:
(1) (Z)-7-(5-benzylene-2,4-dioxothiazolidin-3-yl)-N-hydroxyheptanamide;
(2) (Z)-7-(5-(2-chlorobenzylene)-2,4-dioxathiazolidin-3-yl)-N-hydroxyheptanamide;
(3) (Z)-7-(5-(3-chlorobenzylene)-2,4-dioxathiazolidin-3-yl)-N-hydroxyheptanamide;
(4) (Z)-7-(5-(4-chlorobenzylene)-2,4-dioxathiazolidin-3-yl)-N-hydroxyheptanamide;
(5) (Z)-7-(5-(2-bromobenzylene)-2,4-dioxathiazolidin-3-yl)-N-hydroxyheptanamide;
(6) (Z)-7-(5-(3-bromobenzylene)-2,4-dioxathiazolidin-3-yl)-N-hydroxyheptanamide;
(7) (Z)-7-(5-(4-bromobenzylene)-2,4-dioxathiazolidin-3-yl)-N-hydroxyheptanamide;
(8) (Z)-7-(5-(4-fluorobenzylene)-2,4-dioxathiazolidin-3-yl)-N-hydroxyheptanamide;
(9) (Z)-N-hydroxy-7-(5-(3-methoxybenzylene)-2,4-dioxathiazolidin-3-yl)heptanamide;
(10) (Z)-N-hydroxy-7-(5-(4-methoxybenzylene)-2,4-dioxathiazolidin-3-yl)heptanamide;
(11) methyl (Z)-3-((3-(7-(hydroxyamino)-7-oxoheptyl)-2,4-dioxathiazolidin-5-ylidene)methyl)benzoate;
(12) methyl (Z)-4-((3-(7-(hydroxyamino)-7-oxoheptyl)-2,4-dioxathiazolidin-5-ylidene)methyl)benzoate;
(13) (Z)-7-(5-(4-aminobenzylene)-2,4-dioxathiazolidin-3-yl)-N-hydroxyheptanamide;
(14) (Z)-7-(5-(2-ethoxybenzylene)-2,4-dioxathiazolidin-3-yl)-N-hydroxyheptanamide;
(15) (Z)-N-hydroxy-7-(5-(4-isopropylbenzylene)-2,4-dioxathiazolidin-3-yl)heptanamide;
(16) (Z)-7-(5-(4-bromo-2-methoxybenzylene)-2,4-dioxathiazolidin-3-yl)-N-hydroxyheptanamide;
(17) (Z)-7-(5-(5-bromo-2-methoxybenzylene)-2,4-dioxathiazolidin-3-yl)-N-hydroxyheptanamide;
(18) (Z)-7-(5-(3-bromo-2-methoxybenzylene)-2,4-dioxathiazolidin-3-yl)-N-hydroxyheptanamide;
(19) (Z)-7-(5-(2-bromo-4-methoxybenzylene)-2,4-dioxathiazolidin-3-yl)-N-hydroxyheptanamide;
(20) (Z)-7-(5-(3-bromo-4-methoxybenzylene)-2,4-dioxathiazolidin-3-yl)-N-hydroxyheptanamide;
(21) (Z)-7-(5-(3-chloro-2-methoxybenzylene)-2,4-dioxathiazolidin-3-yl)-N-hydroxyheptanamide;
(22) (Z)-7-(5-(4-chloro-2-methoxybenzylene)-2,4-dioxathiazolidin-3-yl)-N-hydroxyheptanamide;
(23) (Z)-7-(5-(5-chloro-2-methoxybenzylene)-2,4-dioxathiazolidin-3-yl)-N-hydroxyheptanamide;
(24) (Z)-7-(5-(6-chloro-2-methoxybenzylene)-2,4-dioxathiazolidin-3-yl)-N-hydroxyheptanamide;
(25) (Z)-N-hydroxy-7-(5-(2-methoxy-4-methylbenzylene)-2,4-dioxathiazolidin-3-yl)heptanamide;
(26) (Z)-7-(5-(4-fluoro-2-methoxybenzylene)-2,4-dioxathiazolidin-3-yl)-N-hydroxyheptanamide;
(27) (Z)-7-(5-(2,4-dimethoxybenzylene)-2,4-dioxathiazolidin-3-yl)-N-hydroxyheptanamide;
(28) (Z)-N-hydroxy-7-(5-(naphth-2-ylmethylene)-2,4-dioxathiazolidin-3-yl)heptanamide;
(29) (Z)-7-(2,4-dioxo-5-(quinol-2-ylmethylene)thiazolidin-3-yl)-N-hydroxyheptanamide;
(30) (Z)-7-(2,4-dioxo-5-(quinol-7-methylene)thiazolidin-3-yl)-N-hydroxyheptanamide;
(31) (Z)-7-(2,4-dioxo-5-(quinol-3-ylmethylene)thiazolidin-3-yl)-N-hydroxyheptanamide;
(32) (Z)-7-(2,4-dioxy-5-(quinol-6-ylmethylene)thiazolidin-3-yl)-N-hydroxyheptanamide;
(33) (Z)-7-(5-((2,3-dihydrobenzofuran-5-yl)methylene)-2,4-dioxathiazolidin-3-yl)-N-hydroxyheptanamide;
(34) (Z)-7-(5-(benzo[d][1,3]dioxazol5-ylmethylene)-2,4-dioxathiazolin-3-yl)-N-hydroxyheptanamide;
(35) (Z)-7-(2,4-dioxy-5-(pyridin-3-ylmethylene)thiazolidin-3-yl)-N-hydroxyheptanamide;
(36) (Z)-7-(2,4-dioxo-5-(quinol-8-methylene)thiazolidin-3-yl)-N-hydroxyheptanamide;
(37) (Z)-7-(2,4-dioxo-5-(thien-2-ylmethylene)thiazolidin-3-yl)-N-hydroxyheptanamide;
(38) (Z)-7-(5-(furan-2-ylmethylene)-2,4-dioxathiazolidin-3-yl)-N-hydroxyheptanamide;
(39) (Z)-N-hydroxy-7-(5-(4-methoxybenzylene)-4-oxo-2-thiothiazolidin-3-yl)heptanamide;
(40) (Z)-7-(5-(4-chlorobenzylene)-4-oxo-2-thiathiazolidin-3-yl)-N-hydroxyheptanamide;
(41) (Z)-N-hydroxy-7-(5-(4-methylbenzylene)-4-oxo-2-thiothiazolidin-3-yl)heptanamide;
(42) (Z)-7-(5-(4-ethylbenzylene)-4-oxo-2-thiothiazolidin-3-yl)-N-hydroxyheptanamide; and
(43) (Z)-N-(2-amino-4-fluorophenyl)-4-((2,4-dioxy-5-(pyridin-3-ylmethylene)thiazolidin-3-yl)methyl)benzamide.

The present invention further provides a method for preparing the inhibitor described above, wherein a method for preparing thiazolidinedione-based HDAC inhibitors (1-15) includes: subjecting substituted benzaldehydes (1a-1o) and thiazolidinedione, as starting materials, to a Knoevenagel condensation reaction under the action of β-alanine to obtain intermediates (2a-2o), respectively; subjecting the intermediates (2a-2o) and ethyl 7-bromoheptanoate to a nucleophilic substitution reaction to obtain key intermediates (3a-3o), respectively; hydrolyzing the intermediates (3a-3o) under acidic conditions to obtain intermediates (4a-4o), respectively; and condensing the intermediates (4a-4o) with hydroxylamine hydrochloride to obtain target compounds (1-15), respectively. Specifically, thiazolidinone (5 mmol), each of the compounds 1a-1o (5 mmol), and β-alanine (6 mmol) are weighed into 10 mL of glacial acetic acid, and refluxed with stirring for approximately 5 h; after the full consumption of the starting material thiazolidinone is confirmed by thin-layer chromatography (TLC), a reaction mixture is quenched with water and filtered to subsequently collect a filter cake; and the filter cake is dried to obtain compounds 2a-2o, respectively, with the yield of 80.1%. Then, each of the compounds 2a-2o (4 mmol) is added to a round-bottom flask; 10 mL of DMF is added as a solvent, and 1.5 eq. of potassium carbonate (6 mmol) is further added; a resulting mixture is pre-stirred for 30 min in an ice bath; the ice bath is removed, allowing the temperature to rise to room temperature during reaction; 2 eq. of brominated acid ester is added; the mixture is stirred to allow a reaction to proceed for 8 h; then, the reaction mixture is extracted with water and ethyl acetate; and organic layers are collected, concentrated, and eluted and separated by column chromatography using a mixed solvent (petroleum ether: ethyl acetate = 3:1) as an eluent to obtain compounds 3a-3o as a transparent oily liquid, with the yield of 35%-56.2%. 3 mL of glacial acetic acid and 0.5 mL of concentrated hydrochloric acid (10 M) are added to each of the compounds 3a-3o (2 mmol); a resulting mixture is heated to 90°C and stirred for reaction, with a reaction process monitored by TLC until full consumption of the starting materials; the reaction mixture is cooled, followed by slow dropwise addition of a saturated aqueous sodium chloride solution for precipitation of solids; the reaction mixture is filtered; and a filter cake is washed three times with water and dried to obtain compounds 4a-4o, respectively. Finally, each of the compounds 4a-4o from the previous step is directly dissolved in 5 mL of tetrahydrofuan, and stirred with the portionwise addition of CDI (N,N-carbonyldiimidazole, 3 mmol); a resulting mixture is pre-stirred for 1 h; after the full consumption of starting materials is confirmed by TLC, hydroxylamine hydrochloride (6 mmol) is added; the reaction mixture is stirred to allow a reaction to proceed for 12 h overnight, concentrated, and eluted and separated by column chromatography using a mixed solvent (dichloromethane: methanol = 25:1) as an eluent to obtain compounds 5a-5o, with the yield of 11%-20.2%. A preparation route is as follows:

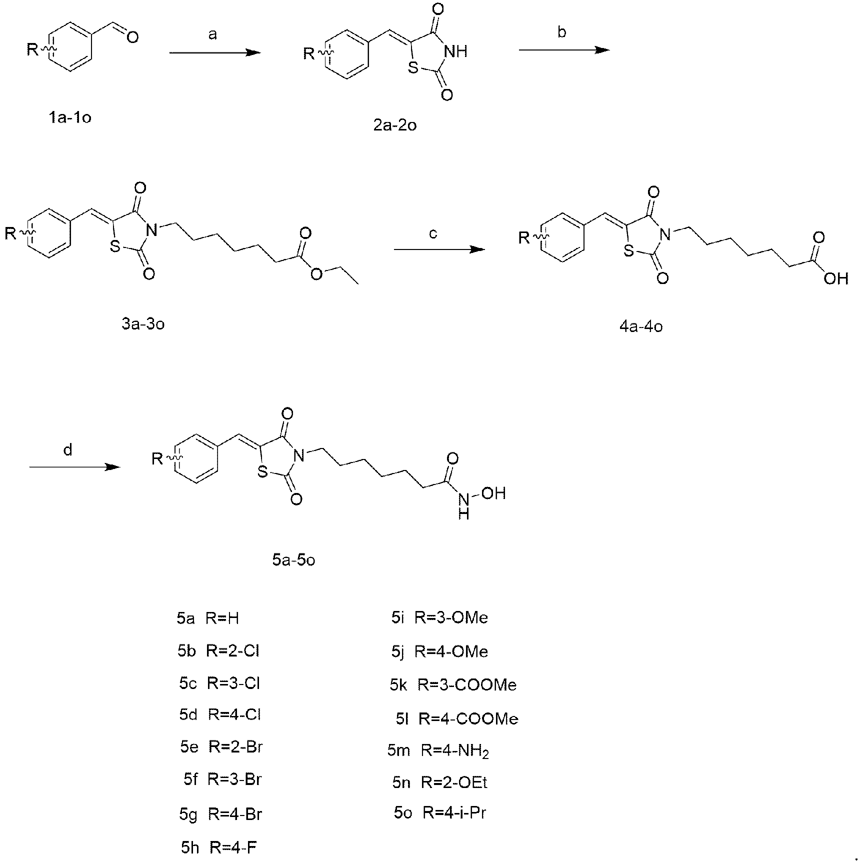

A method for preparing thiazolidinedione-based HDAC inhibitors (16-27) includes: subjecting poly-substituted benzaldehydes (6a-6I) and thiazolidinedione, as starting materials, to a Knoevenagel condensation reaction under the action of β-alanine to obtain intermediates (7a-7I), respectively; subjecting the intermediates (7a-7I) and ethyl 7-bromoheptanoate to a nucleophilic substitution reaction to obtain key intermediates (8a-8I), respectively; hydrolyzing the intermediates (8a-8I) under acidic conditions to obtain intermediates (9a-9I), respectively; and condensing the intermediates (9a-9I) with hydroxylamine hydrochloride to obtain target compounds (16-27), respectively. Specifically, thiazolidinone (5 mmol), each of the compounds 6a-6I (5 mmol), and β-alanine (6 mmol) are weighed into 10 mL of glacial acetic acid, and refluxed with stirring for approximately 5 h; after the full consumption of the starting material thiazolidinone is confirmed by TLC, a reaction mixture is quenched with water and filtered to subsequently collect a filter cake; and the filter cake is dried to obtain compounds 7a-7I, respectively, with the yield of 80.1%. Then, each of the compounds 7a-7I (4 mmol) is added to a round-bottom flask; 10 mL of DMF is added as a solvent, and 1.5 eq. of potassium carbonate (6 mmol) is further added; a resulting mixture is pre-stirred for 30 min in an ice bath; the ice bath is removed, allowing the temperature to rise to room temperature during reaction; 2 eq. of brominated acid ester is added; the mixture is stirred to allow a reaction to proceed for 8 h; then, the reaction mixture is extracted with water and ethyl acetate; and organic layers are collected, concentrated, and eluted and separated by column chromatography using a mixed solvent (petroleum ether: ethyl acetate = 3:1) as an eluent to obtain compounds 8a-8I as a transparent oily liquid, with the yield of 35%-56.2%. 3 mL of glacial acetic acid and 0.5 mL of concentrated hydrochloric acid (10 M) are added to each of the compounds 8a-8I (2 mmol); a resulting mixture is heated to 90°C and stirred for reaction, with a reaction process monitored by TLC until full consumption of the starting materials; the reaction mixture is cooled, followed by slow dropwise addition of a saturated aqueous sodium chloride solution for precipitation of solids; the reaction mixture is filtered; and a filter cake is washed three times with water and dried to obtain compounds 9a-9I, respectively. Finally, each of the compounds 9a-9I from the previous step is directly dissolved in 5 mL of tetrahydrofuan, and stirred with the portionwise addition of CDI (N,N-carbonyldiimidazole, 3 mmol); a resulting mixture is pre-stirred for 1 h; after the full consumption of starting materials is confirmed by TLC, hydroxylamine hydrochloride (6 mmol) is added; the reaction mixture is stirred to allow a reaction to proceed for 12 h overnight, concentrated, and eluted and separated by column chromatography using a mixed solvent (dichloromethane: methanol = 25:1) as an eluent to obtain compounds 10a-10I, with the yield of 11%-20.2%. A preparation route is as follows:

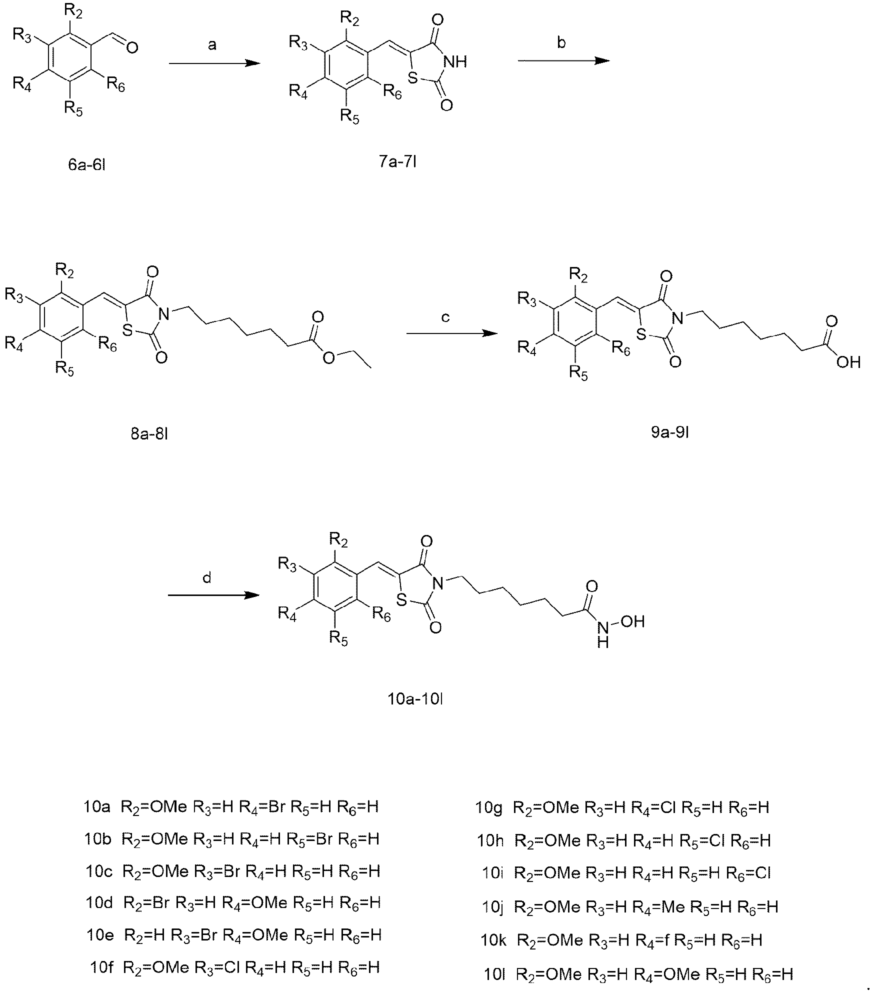

A method for preparing thiazolidinedione-based HDAC inhibitors (28-38) includes: subjecting aromatic aldehydes (11a-11k) and thiazolidinedione, as starting materials, to a Knoevenagel condensation reaction under the action of β-alanine to obtain intermediates (12a-12k), respectively; subjecting the intermediates (12a-12k) and ethyl 7-bromoheptanoate to a nucleophilic substitution reaction to obtain key intermediates (13a-13k), respectively; hydrolyzing the intermediates (13a-13k) under acidic conditions to obtain intermediates (14a-14k), respectively; and condensing the intermediates (14a-14k) with hydroxylamine hydrochloride to obtain target compounds (28-38), respectively. Specifically, thiazolidinone (5 mmol), each of the compounds 11a-11k (5 mmol), and β-alanine (6 mmol) are weighed into 10 mL of glacial acetic acid, and refluxed with stirring for approximately 5 h; after the full consumption of the starting material thiazolidinone is confirmed by TLC, a reaction mixture is quenched with water and filtered to subsequently collect a filter cake; and the filter cake is dried to obtain compounds 12a-12k, respectively, with the yield of 80.1%. Then, each of the compounds 12a-12k (4 mmol) is added to a round-bottom flask; 10 mL of DMF is added as a solvent, and 1.5 eq. of potassium carbonate (6 mmol) is further added; a resulting mixture is pre-stirred for 30 min in an ice bath; the ice bath is removed, allowing the temperature to rise to room temperature during reaction; 2 eq. of brominated acid ester is added; the mixture is stirred to allow a reaction to proceed for 8 h; then, the reaction mixture is extracted with water and ethyl acetate; and organic layers are collected, concentrated, and eluted and separated by column chromatography using a mixed solvent (petroleum ether: ethyl acetate = 3:1) as an eluent to obtain compounds 13a-13k as a transparent oily liquid, with the yield of 35%-56.2%. 3 mL of glacial acetic acid and 0.5 mL of concentrated hydrochloric acid (10 M) are added to each of the compounds 13a-13k (2 mmol); a resulting mixture is heated to 90°C and stirred for reaction, with a reaction process monitored by TLC until full consumption of the starting materials; the reaction mixture is cooled, followed by slow dropwise addition of a saturated aqueous sodium chloride solution for precipitation of solids; the reaction mixture is filtered; and a filter cake is washed three times with water and dried to obtain compounds 14a-14k, respectively. Finally, each of the compounds 14a-14k from the previous step is directly dissolved in 5 mL of tetrahydrofuan, and stirred with the portionwise addition of CDI (N,N-carbonyldiimidazole, 3 mmol); a resulting mixture is pre-stirred for 1 h; after the full consumption of starting materials is confirmed by TLC, hydroxylamine hydrochloride (6 mmol) is added; the reaction mixture is stirred to allow a reaction to proceed for 12 h overnight, concentrated, and eluted and separated by column chromatography using a mixed solvent (dichloromethane: methanol = 25:1) as an eluent to obtain compounds 15a-15k, with the yield of 11%-20.2%. A preparation route is as follows:

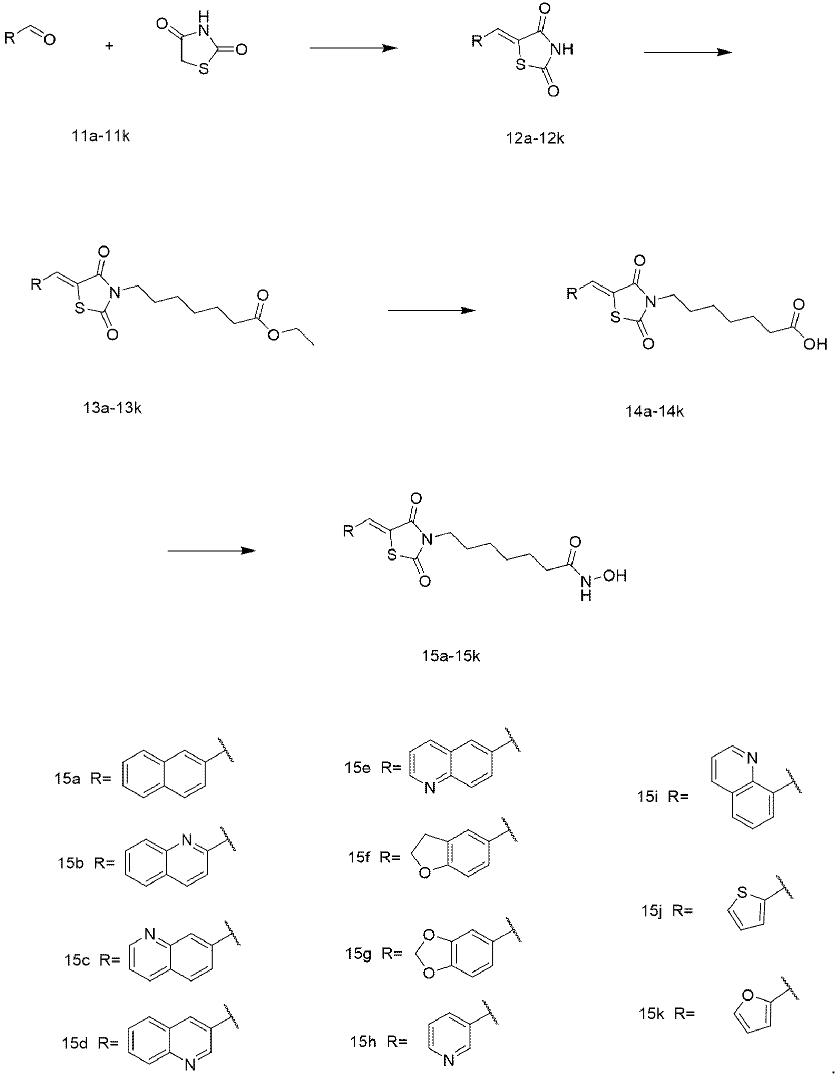

A method for preparing thiazolidinedione-based HDAC inhibitors (39-42) includes: subjecting para-substituted benzaldehydes (16a-16d) and rhodanine, as starting materials, to a Knoevenagel condensation reaction under the action of β-alanine to obtain intermediates (17a-17d), respectively; subjecting the intermediates (17a-17d) and ethyl 7-bromoheptanoate to a nucleophilic substitution reaction to obtain key intermediates (18a-18d), respectively; hydrolyzing the intermediates (18a-18d) under acidic conditions to obtain intermediates (19a-19d), respectively; and condensing the intermediates (19a-19d) with hydroxylamine hydrochloride to obtain target compounds (39-42), respectively. Specifically, rhodanine (5 mmol), each of the compounds 16a-16d (5 mmol), and β-alanine (6 mmol) are weighed into 10 mL of glacial acetic acid, and refluxed with stirring for approximately 5 h; after the full consumption of the starting material rhodaninethiazolidinone is confirmed by TLC, a reaction mixture is quenched with water and filtered to subsequently collect a filter cake; and the filter cake is dried to obtain compounds 17a-17d, respectively, with the yield of 80.1%. Then, each of the compounds 17a-17d (4 mmol) is added to a round-bottom flask; 10 mL of DMF is added as a solvent, and 1.5 eq. of potassium carbonate (6 mmol) is further added; a resulting mixture is pre-stirred for 30min in an ice bath; the ice bath is removed, allowing the temperature to rise to room temperature during reaction; 2 eq. of brominated acid ester is added; the mixture is stirred to allow a reaction to proceed for 8 h; then, the reaction mixture is extracted with water and ethyl acetate; and organic layers are collected, concentrated, and eluted and separated by column chromatography using a mixed solvent (petroleum ether: ethyl acetate = 3:1) as an eluent to obtain compounds 18a-18d as a transparent oily liquid, with the yield of 35%-56.2%. 3 mL of glacial acetic acid and 0.5 mL of concentrated hydrochloric acid (10 M) are added to each of the compounds 18a-18d (2 mmol); a resulting mixture is heated to 90°C and stirred for reaction, with a reaction process monitored by TLC until full consumption of the starting materials; the reaction mixture is cooled, followed by slow dropwise addition of a saturated aqueous sodium chloride solution for precipitation of solids; the reaction mixture is filtered; and a filter cake is washed three times with water and dried to obtain compounds 19a-19d, respectively. Finally, each of the compounds 19a-19d from the previous step is directly dissolved in 5 mL of tetrahydrofuan, and stirred with the portionwise addition of CDI (N,N-carbonyldiimidazole, 3 mmol); a resulting mixture is pre-stirred for 1 h; after the full consumption of starting materials is confirmed by TLC, hydroxylamine hydrochloride (6 mmol) is added; the reaction mixture is stirred to allow a reaction to proceed for 12 h overnight, concentrated, and eluted and separated by column chromatography using a mixed solvent (dichloromethane: methanol = 25:1) as an eluent to obtain compounds 20a-20d, with the yield of 11%-20.2%. A preparation route is as follows:

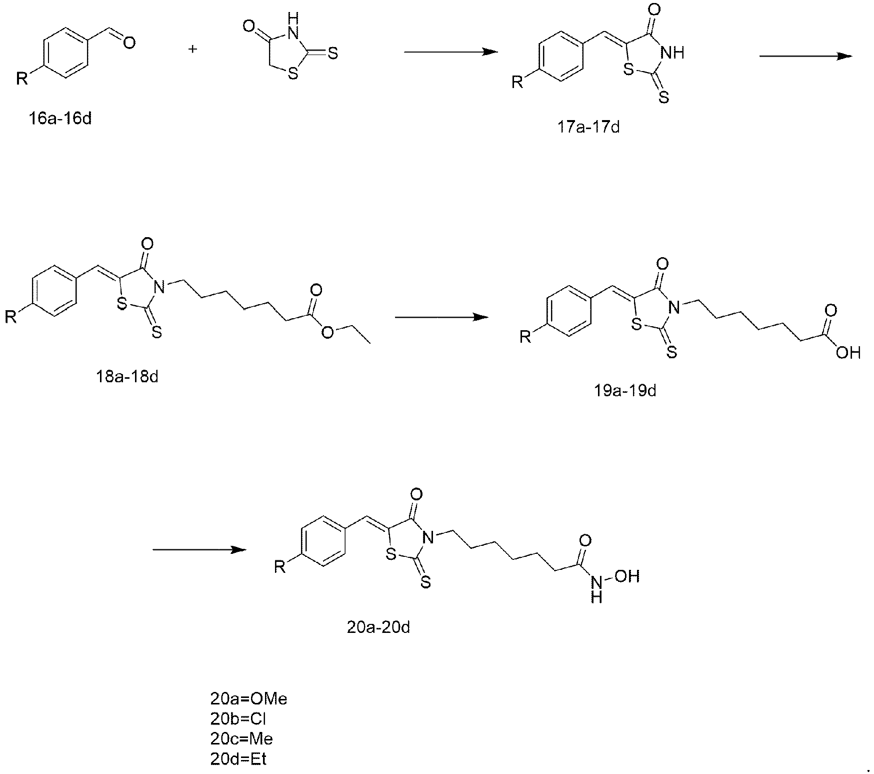

The present invention further provides use of the aforementioned thiazolidinedione-based HDAC inhibitor or the aforementioned pharmaceutical composition in the preparation of a medicament for in vitro inhibition of cancer cell growth.

The compound of the present invention has a novel structure and serves as an efficient HDAC inhibitor, with the beneficial effects of efficient and excellent HDAC inhibitory activity, low cost, good efficacy, low toxicity and high yields of intermediates during synthesis, resulting in reduced resource waste and further reduced cost. The present invention can be applied to the research of related anti-tumor drugs, and has positive and predictable clinical application value for anti-tumor drugs, as well as a very promising development prospect.

### SPECIFIC IMPLEMENTATIONS

The technical solutions of the present invention will be further defined below in conjunction with specific embodiments, and the scope of protection as claimed is not limited to the description. In the examples described below, all temperatures were set in degrees Celsius, unless otherwise indicated. Reagents were commercially available from commercial suppliers such as Anhui Zesheng Technology Co., Ltd., J&K Scientific, Aladdin Reagent Co.,Ltd., Beijing Ouhe Technology Co., Ltd., etc., and were used without further purification, unless otherwise indicated. Common reagents were commercially available from Xilong Chemical Co., Ltd., Nanjing Chemical Reagent Co., Ltd., Sinopharm Chemical Reagent Co., Ltd., Qingdao Haiyang Chemical Co., Ltd., etc.

In the examples described below, chromatographic purification was performed using silica gel columns (200-300 mesh) commercially available from Qingdao Haiyang Chemical Co., Ltd. Nuclear magnetic resonance (NMR) spectra were recorded in a CDCl₃ or DMSO-*d*₆ solvent (in ppm), using tetramethylsilane (TMS) at 0 ppm as internal reference. Peak multiplicities were denoted in abbreviation as follows: s (singlet), d (doublet), t (triplet), m (multiplet), br (broadened), dd (doublet of doublets), dt (doublet of triplets). Coupling constants were expressed in hertz (Hz).

In the examples described below, for ease of description, some starting materials will be described by their abbreviations, which are illustrated below in contrast with their full names: DCM denotes CH₂Cl₂, i.e., dichloromethane; CDCl₃ denotes deuterated chloroform; PE denotes petroleum ether; both EtOAc and EA denote ethyl acetate; both MeOH and CH₃OH denote methanol; ClSO₃H denotes chlorosulfonic acid; both TEA and Et₃N denote triethylamine; DMSO-d₆ denotes hexadeuterated dimethyl sulfoxide; THF denotes tetrahydrofuran; NaCl denotes sodium chloride; Na₂SO₄ denotes sodium sulfate; and CDI denotes N,N-carbonyldiimidazole.

### Example 1

Synthesis of (Z)-7-(5-benzylene-2,4-dioxothiazolidin-3-yl)-N-hydroxyheptanamide, with synthesis steps as below. Step 1: Synthesis of (Z)-5-benzylenethiazolidin-2,4-dione, with a structural formula as below: 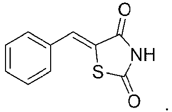 Thiazolidinone (5 mmol, 585.6 mg), acetic acid (10mL) and benzaldehyde (5 mmol, 530.6 mg) were sequentially added to a 100 mL round-bottom flask and refluxed with stirring at 120°C to allow a reaction to proceed for 8 h. A reaction mixture was cooled to room temperature for precipitation of solids, and then subjected to suction filtration; and a filter cake was dried to obtain 820 mg of white solid, with the yield of 80.1%.

Step 2: Synthesis of ethyl (Z)-7-(5-benzylene-2,4-dioxothiazolidin-3-yl)heptanate, with a structural formula as below: 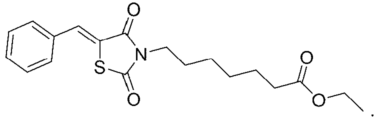 4 mmol (Z)-5-benzylenethiazol-2,4-dione was added to a round-bottom flask; 10 mL of DMF was added as a solvent, and 1.5 eq. of potassium carbonate (6 mmol) was further added; a resulting mixture was pre-stirred for 30 min in an ice bath; the ice bath was removed, allowing the temperature to rise to room temperature during reaction; 2 eq. of brominated acid ester was added; the mixture was stirred to allow a reaction to proceed for 8 h; then, the reaction mixture was extracted with water and ethyl acetate; and organic layers were collected, concentrated, and eluted and separated by column chromatography using a mixed solvent (petroleum ether:ethyl acetate = 3:1 by volume) as an eluent to obtain a compound ethyl (Z)-7-(5-benzylene-2,4-dioxothiazolidin-3-yl)heptanate as a transparent oily liquid, with the yield of 35%-56.2%.

Step 3: Synthesis of (Z)-7-(5-benzylene-2,4-dioxothiazolidin-3-yl)heptanoic acid, with a structural formula as below: 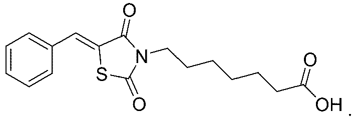 3 mL of glacial acetic acid and 0.5 mL of concentrated hydrochloric acid (10 M) were added to the compound ethyl (Z)-7-(5-benzylene-2,4-dioxothiazolidin-3-yl)heptanate (2 mmol); a resulting mixture was heated to 90°C and stirred for reaction, with a reaction process monitored by TLC until full consumption of the starting materials; the reaction mixture was cooled, followed by slow dropwise addition of saturated aqueous sodium chloride solution for precipitation of solids; the reaction mixture was filtered; and a filter cake was washed three times with water and dried to obtain crude (Z)-7-(5-benzylene-2,4-dioxothiazolidin-3-yl)heptanoic acid.

Step 4: Synthesis of (Z)-7-(5-benzylene-2,4-dioxothiazolidin-3-yl)-N-hydroxyheptanamide, with a structural formula as below: 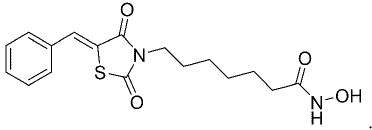 (Z)-7-(5-benzylene-2,4-dioxothiazolidin-3-yl)heptanoic acid from the previous step was dissolved in 5 mL of tetrahydrofuan, and stirred with the portionwise addition of CDI (N,N-carbonyldiimidazole, 3 mmol); a resulting mixture was pre-stirred for 1 h; after the full consumption of starting materials was confirmed by TLC, hydroxylamine hydrochloride (6 mmol) was added; the reaction mixture was stirred to allow a reaction to proceed for 12 h overnight, concentrated, and eluted and separated by column chromatography using a mixed solvent (dichloromethane:methanol = 25:1 by volume) as an eluent to obtain (Z)-7-(5-benzylene-2,4-dioxothiazolidin-3-yl)-N-hydroxyheptanamide, with the yield of 11%-20.2%. ¹H NMR (400 MHz, DMSO-*d*₆) δ 10.34 (s, 1H), 8.66 (s, 1H), 7.93 (s, 1H), 7.63 (d,*J* = 7.0 Hz, 2H), 7.53 (dt,*J* = 13.0, 6.8 Hz, 3H), 3.64 (t,*J* = 7.2 Hz, 2H), 1.93 (t,*J* = 7.3 Hz, 2H), 1.57 (t,*J* = 6.8 Hz, 2H), 1.48 (s, 2H), 1.54 - 1.42 (m, 1H), 1.30 - 1.21 (m, 4H). ¹³C NMR (101 MHz, DMSO-*d*₆) δ 169.51, 167.80, 166.13, 133.45, 133.30, 131.08, 130.57, 129.84, 121.83, 41.98, 32.61, 28.55, 27.44, 26.30, 25.41.

### Example 2

Synthesis of (Z)-7-(5-(2-chlorobenzylene)-2.4-dioxathiazolidin-3-yl)-N-hydroxyheptanamide, with a structural formula as below: 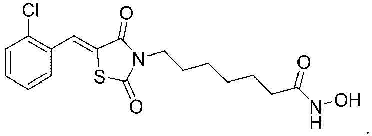 Synthesis method: the fragment (Z)-7-(5-benzylene-2,4-dioxothiazolidin-3-yl)heptylic acid in Step 4 of Example 1 was replaced with (Z)-7-(5-(2-chlorobenzylene)-2,4-dioxathiazolidin-3-yl)heptylic acid, while other steps and procedures were identical to those in Example 1. White solid, yield: 17%. ¹H NMR (300 MHz, DMSO-*d*₆) δ 10.36 (s, 1H), 8.69 (s, 1H), 8.04 (s, 1H), 7.66 (dt,*J* = 7.8, 2.9 Hz, 1H), 7.66 - 7.46 (m, 3H), 3.64 (t,*J* = 7.2 Hz, 2H), 2.02 - 1.88 (m, 2H), 1.52 (dt,*J* = 28.4, 6.9 Hz, 2H), 1.32 - 1.12 (m, 4H).

### Example 3

(Z)-7-(5-(3-chlorobenzylene)-2,4-dioxathiazolidin-3-yl)-N-hydroxyheptanamide, with a structural formula as below: 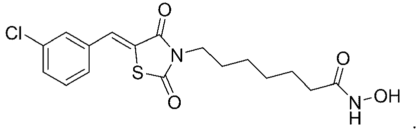 Synthesis method: the fragment (Z)-7-(5-benzylene-2,4-dioxothiazolidin-3-yl)heptylic acid in Step 4 of Example 1 was replaced with (Z)-7-(5-(3-chlorobenzylene)-2,4-dioxathiazolidin-3-yl)heptylic acid, while other steps and procedures were identical to those in Example 1. White solid, yield: 14%. ¹H NMR (400 MHz, DMSO-*d*₆) δ 10.33 (s, 1H), 8.65 (s, 1H), 7.92 (s, 1H), 7.72 (s, 1H), 7.57 (s, 3H), 3.64 (t,*J* = 7.2 Hz, 2H), 2.25 (s, 0H), 1.93 (t,*J* = 7.4 Hz, 2H), 1.57 (p,*J* = 6.8 Hz, 2H), 1.47 (q,*J*= 7.0 Hz, 2H), 1.30 - 1.21 (m, 4H). ¹³C NMR (101 MHz, DMSO-*d*₆) δ 169.48, 167.46, 165.92, 135.64, 134.44, 131.65, 130.64, 130.50, 128.26, 123.67, 42.08, 32.61, 28.55, 27.41, 26.30, 25.41.

### Example 4

(Z) -7-(5-(4-chlorobenzylene)-2,4-dioxathiazolidin-3-yl)-N-hydroxyheptanamide, with a structural formula as below: 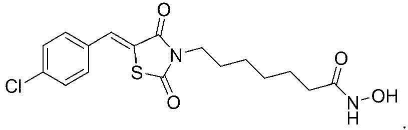 Synthesis method: the fragment (Z)-7-(5-benzylene-2,4-dioxothiazolidin-3-yl)heptylic acid in Step 4 of Example 1 was replaced with (Z)-7-(5-(4-chlorobenzylene)-2,4-dioxathiazolidin-3-yl)heptylic acid, while other steps and procedures were identical to those in Example 1. White solid, yield: 15%. ¹H NMR (400 MHz, DMSO-*d*₆) δ 10.33 (s, 1H), 8.66 (s, 1H), 7.93 (s, 1H), 7.63 (q,*J* = 8.5 Hz, 4H), 3.64 (t,*J* = 7.2 Hz, 2H), 2.25 (s, 0H), 1.93 (t,*J* = 7.4 Hz, 2H), 1.57 (p,*J* = 6.9 Hz, 2H), 1.47 (t,*J* = 7.2 Hz, 2H), 1.26 (qd,*J* = 7.6, 4.9, 3.7 Hz, 4H), 0.78 (s, 0H). ¹³C NMR (101 MHz, DMSO-*d*₆) δ 169.49, 167.55, 166.03, 135.66, 132.37, 132.20, 131.96, 129.91, 122.62, 42.04, 32.61, 28.55, 27.42, 26.29, 25.40.

### Example 5

(Z)-7-(5-(2-bromobenzylene)-2,4-dioxathiazolidin-3-yl)-N-hydroxyheptanamide, with a structural formula as below: 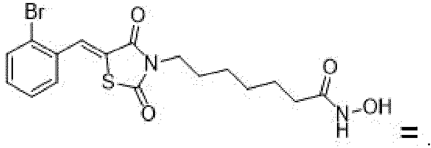 Synthesis method: the fragment (Z)-7-(5-benzylene-2,4-dioxothiazolidin-3-yl)heptylic acid in Step 4 of Example 1 was replaced with (Z)-7-(5-(2-bromobenzylene)-2,4-dioxathiazolidin-3-yl)heptylic acid, while other steps and procedures were identical to those in Example 1. White solid, yield: 14%. ¹H NMR (400 MHz, DMSO-*d*₆) δ 10.34 (s, 1H), 8.66 (s, 1H), 8.00 (s, 1H), 7.83 (d,*J* = 7.9 Hz, 1H), 7.59 (q,*J* = 3.8, 2.9 Hz, 2H), 7.43 (ddd,*J* = 8.5, 5.9, 3.1 Hz, 1H), 3.68 - 3.58 (m, 2H), 2.26 (s, 0H), 1.94 (t,*J* = 7.4 Hz, 2H), 1.70 (p,*J* = 6.8 Hz, 0H), 1.58 (d,*J* = 14.5 Hz, 0H), 1.58 (s, 2H), 1.48 (t,*J* = 7.2 Hz, 2H), 1.43 - 1.32 (m, 0H), 1.27 (p,*J* = 3.5 Hz, 4H). ¹³C NMR (101 MHz, DMSO-*d*₆) δ 169.49, 167.63, 165.74, 134.11, 133.19, 132.62, 130.94, 129.57, 129.14, 125.76, 125.64, 42.14, 32.62, 28.55, 27.40, 26.31, 25.40.

### Example 6

(Z)-7-(5-(3-bromobenzylene)-2,4-dioxathiazolidin-3-yl)-N-hydroxyheptanamide, with a structural formula as below: 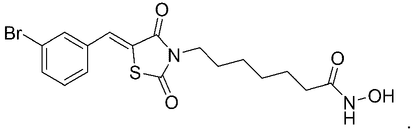 Synthesis method: the fragment (Z)-7-(5-benzylene-2,4-dioxothiazolidin-3-yl)heptylic acid in Step 4 of Example 1 was replaced with (Z)-7-(5-(3-bromobenzylene)-2,4-dioxathiazolidin-3-yl)heptylic acid, while other steps and procedures were identical to those in Example 1. White solid, yield: 21%. ¹H NMR (400 MHz, DMSO-*d*₆) δ 10.33 (s, 1H), 8.66 (s, 1H), 7.91 (s, 1H), 7.85 (d,*J* = 1.8 Hz, 1H), 7.69 (dt,*J* = 8.0, 1.3 Hz, 1H), 7.64 - 7.57 (m, 1H), 7.51 (t,*J* = 7.9 Hz, 1H), 3.64 (t,*J* = 7.2 Hz, 2H), 2.25 (s, 0H), 1.93 (t,*J* = 7.4 Hz, 2H), 1.52 (dt,*J* = 36.6, 7.2 Hz, 5H), 1.25 (td,*J* = 8.2, 7.7, 4.0 Hz, 5H), 0.79 (s, 0H). ¹³C NMR (101 MHz, DMSO-*d*₆) δ 169.49, 167.45, 165.90, 135.89, 133.52, 133.39, 131.85, 131.61, 128.59, 123.63, 122.95, 42.08, 32.61, 28.55, 27.41, 26.30, 25.41.

### Example 7

(Z)-7-(5-(4-bromobenzylene)-2,4-dioxathiazolidin-3-yl)-N-hydroxyheptanamide, with a structural formula as below: 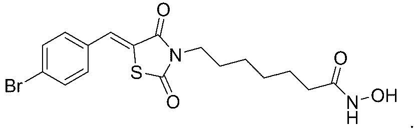 Synthesis method: the fragment (Z)-7-(5-benzylene-2,4-dioxothiazolidin-3-yl)heptylic acid in Step 4 of Example 1 was replaced with (Z)-7-(5-(4-bromobenzylene)-2,4-dioxathiazolidin-3-yl)heptylic acid, while other steps and procedures were identical to those in Example 1. White solid, yield: 18%. ¹H NMR (400 MHz, DMSO-*d*₆) δ 10.33 (s, 1H), 8.66 (d,*J* = 1.4 Hz, 1H), 7.90 (s, 1H), 7.75 (d,*J* = 8.4 Hz, 2H), 7.57 (d,*J* = 8.3 Hz, 2H), 3.63 (t,*J* = 7.2 Hz, 2H), 2.25 (s, 0H), 1.93 (t,*J* = 7.4 Hz, 2H), 1.56 (t,*J* = 7.0 Hz, 2H), 1.47 (s, 2H), 1.53 - 1.41 (m, 1H), 1.32 - 1.21 (m, 4H). ¹³C NMR (101 MHz, DMSO-*d*₆) δ 169.49, 167.53, 166.03, 132.83, 132.68, 132.34, 132.05, 124.59, 122.69, 42.05, 32.61, 28.55, 27.42, 26.30, 25.41.

### Example 8

(Z)-7-(5-(4-fluorobenzylene)-2,4-dioxathiazolidin-3-yl)-N-hydroxyheptanamide, with a structural formula as below: 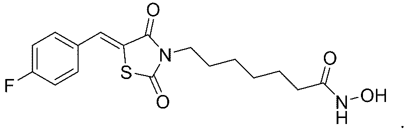 Synthesis method: the fragment (Z)-7-(5-benzylene-2,4-dioxothiazolidin-3-yl)heptylic acid in Step 4 of Example 1 was replaced with (Z)-7-(5-(4-fluorobenzylene)-2,4-dioxathiazolidin-3-yl)heptylic acid, while other steps and procedures were identical to those in Example 1. White solid, yield19%. ¹H NMR (300 MHz, DMSO-*d*₆) δ 10.38 - 10.31 (m, 1H), 8.68 (d,*J* = 1.4 Hz, 1H), 7.94 (s, 1H), 7.78 - 7.64 (m, 2H), 7.40 (t,*J* = 8.8 Hz, 2H), 3.63 (t,*J* = 7.2 Hz, 2H), 2.24 (d,*J* = 7.7 Hz, 0H), 1.93 (t,*J* = 7.3 Hz, 2H), 1.52 (dt,*J* = 26.6, 7.0 Hz, 5H), 1.33 - 1.19 (m, 4H). ¹³C NMR (101 MHz, DMSO-*d*₆) δ 169.49, 167.68, 166.09, 164.67, 162.17, 133.11, 133.02, 132.23, 130.15, 130.12, 121.54, 121.52, 117.13, 116.91, 41.99, 32.61, 28.55, 27.44, 26.30, 25.41.

### Example 9

(Z)-7-(5-(3-methoxybenzylene)-2,4-dioxathiazolidin-3-yl)-N-hydroxyheptanamide, with a structural formula as below: 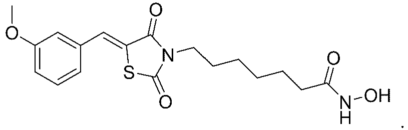 Synthesis method: the fragment (Z)-7-(5-benzylene-2,4-dioxothiazolidin-3-yl)heptylic acid in Step 4 of Example 1 was replaced with (Z)-7-(5-(3-methoxybenzylene)-2,4-dioxathiazolidin-3-yl)heptylic acid, while other steps and procedures were identical to those in Example 1. White solid, yield: 25%. ¹H NMR (400 MHz, DMSO-*d*₆) δ 10.34 (s, 1H), 8.67 (d,*J* = 1.3 Hz, 1H), 7.90 (s, 1H), 7.47 (t,*J* = 8.2 Hz, 1H), 7.22 - 7.16 (m, 2H), 7.08 (ddd,*J* = 8.3, 2.5, 1.0 Hz, 1H), 3.63 (t,*J* = 7.2 Hz, 2H), 3.35 (s, 2H), 2.25 (t,*J* = 7.5 Hz, 0H), 2.02 - 1.89 (m, 2H), 1.56 (p,*J* = 7.0 Hz, 2H), 1.47 (s, 2H), 1.54 - 1.41 (m, 1H), 1.33 - 1.13 (m, 5H). ¹³C NMR (101 MHz, DMSO-*d*₆) δ 169.49, 167.74, 166.07, 160.10, 134.81, 133.26, 130.94, 122.39, 122.17, 116.94, 115.90, 60.23, 55.76, 41.97, 32.61, 28.55, 27.44, 26.30, 25.42, 21.23, 14.55.

### Example 10

(Z)-7-(5-(4-methoxybenzylene)-2,4-dioxathiazolidin-3-yl)-N-hydroxyheptanamide, with a structural formula as below: 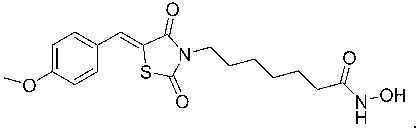 Synthesis method: the fragment (Z)-7-(5-benzylene-2,4-dioxothiazolidin-3-yl)heptylic acid in Step 4 of Example 1 was replaced with (Z)-7-(5-(4-methoxybenzylene)-2,4-dioxathiazolidin-3-yl)heptylic acid, while other steps and procedures were identical to those in Example 1. White solid, yield: 25%. ¹H NMR (300 MHz, DMSO-*d*₆) δ 10.34 (s, 1H), 8.68 (s, 1H), 7.89 (s, 1H), 7.66 - 7.54 (m, 2H), 7.18 - 7.06 (m, 2H), 3.84 (s, 3H), 3.63 (t,*J* = 7.2 Hz, 2H), 1.93 (t,*J* = 7.3 Hz, 2H), 1.59 - 1.42 (m, 1H), 1.31 - 1.19 (m, 4H). ¹³C NMR (101 MHz, DMSO-*d*₆) δ 203.28, 169.49, 167.86, 166.24, 161.61, 133.36, 132.72, 129.32, 125.91, 118.49, 115.44, 60.23, 55.98, 41.87, 32.61, 28.55, 27.47, 26.30, 25.41, 21.23, 14.55.

### Example 11

Methyl (Z)-3-((3-(7-(hydroxyamino)-7-oxoheptyl)-2,4-dioxathiazolidin-5-ylidene)methyl)benzoate, with a structural formula as below: 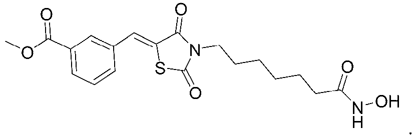 Synthesis method: the fragment (Z)-7-(5-benzylene-2,4-dioxothiazolidin-3-yl)heptylic acid in Step 4 of Example 1 was replaced with (Z)-7-(5-(3-(methoxycarbonyl)benzylene)-2,4-dioxathiazolidin-3-yl)heptylic acid, while other steps and procedures were identical to those in Example 1. White solid, yield: 25%. ¹H NMR (400 MHz, DMSO-*d*₆) δ 10.35 (s, 1H), 8.68 (s, 1H), 8.17 (d,*J* = 1.9 Hz, 1H), 8.11 - 7.98 (m, 2H), 7.95 - 7.85 (m, 1H), 7.69 (t,*J* = 7.8 Hz, 1H). ¹³C NMR (101 MHz, DMSO-*d*₆) δ 169.49, 167.46, 165.98, 165.94, 134.96, 134.06, 132.09, 131.16, 131.05, 130.63, 130.44, 123.30, 55.38, 52.98, 42.07, 32.61, 28.56, 27.42, 26.30, 25.41.

### Example 12

Methyl (Z)-4-((3-(7-(hydroxyamino)-7-oxoheptyl)-2,4-dioxathiazolidin-5-ylidene)methyl)benzoate, with a structural formula as below: 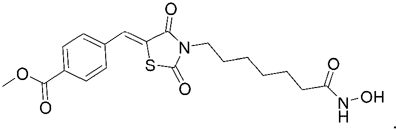 Synthesis method: the fragment (Z)-7-(5-benzylene-2,4-dioxothiazolidin-3-yl)heptylic acid in Step 4 of Example 1 was replaced with (Z)-7-(5-(4-(methoxycarbonyl)benzylene)-2,4-dioxathiazolidin-3-yl)heptylic acid, while other steps and procedures were identical to those in Example 1. White solid, yield: 25%. ¹H NMR (300 MHz, DMSO-*d*₆) δ 10.35 (s, 1H), 8.68 (s, 1H), 8.07 (d,*J* = 8.2 Hz, 2H), 7.97 (s, 1H), 7.76 (d,*J* = 8.1 Hz, 2H), 3.88 (s, 3H), 3.64 (t,*J* = 7.2 Hz, 2H), 2.25 (s, 0H), 1.93 (t,*J* = 7.3 Hz, 2H), 1.52 (dt,*J* = 28.8, 7.1 Hz, 5H), 1.25 (td,*J* = 8.1, 7.5, 3.5 Hz, 4H). ¹³C NMR (101 MHz, DMSO-*d*₆) δ 169.49, 167.54, 165.98, 165.96, 163.01, 137.81, 131.71, 131.01, 130.69, 130.36, 124.56, 60.22, 52.89, 42.10, 32.61, 28.55, 27.41, 26.29, 25.41, 21.23, 14.55.

### Example 13

(Z)-7-(5-(4-aminobenzylene)-2,4-dioxathiazolidin-3-yl)-N-hydroxyheptanamide, with a structural formula as below: 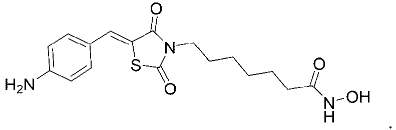 Synthesis method: the fragment (Z)-7-(5-benzylene-2,4-dioxothiazolidin-3-yl)heptylic acid in Step 4 of Example 1 was replaced with (Z)-7-(5-(4-aminobenzylene)-2,4-dioxathiazolidin-3-yl)heptylic acid, while other steps and procedures were identical to those in Example 1. White solid, yield: 25%. ¹H NMR (400 MHz, Acetone-*d*₆) δ 9.88 (s, 1H), 8.21 (s, 1H), 7.27 (s, 1H), 6.91 - 6.83 (m, 2H), 6.26 - 6.17 (m, 2H), 5.70 (s, 2H), 3.20 - 3.12 (m, 2H), 2.75 - 2.70 (m, 0H), 1.79 (d,*J* = 8.1 Hz, 0H), 1.57 - 1.44 (m, 2H), 1.05 (dq,*J* = 29.2, 7.3 Hz, 5H), 0.87 - 0.68 (m, 4H). ¹³C NMR (101 MHz, DMSO-*d*₆) δ 169.49, 168.09, 166.39, 152.52, 134.83, 133.26, 120.17, 114.37, 112.83, 60.22, 41.63, 32.61, 28.56, 27.55, 26.31, 25.42, 21.23, 14.56.

### Example 14

(Z)-7-(5-(2-ethoxybenzylene)-2,4-dioxathiazolidin-3-yl)-N-hydroxyheptanamide, with a structural formula as below: 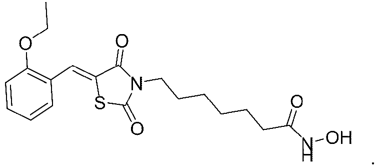 Synthesis method: the fragment (Z)-7-(5-benzylene-2,4-dioxothiazolidin-3-yl)heptylic acid in Step 4 of Example 1 was replaced with (Z)-7-(5-(2-ethoxybenzylene)-2,4-dioxathiazolidin-3-yl)heptylic acid, while other steps and procedures were identical to those in Example 1. White solid, yield: 25%. ¹H NMR (400 MHz, DMSO-*d*₆) δ 10.34 (s, 1H), 8.69 - 8.63 (m, 1H), 8.11 (s, 1H), 7.53 - 7.40 (m, 2H), 7.19 - 7.05 (m, 2H), 4.17 (q,*J* = 6.9 Hz, 2H), 3.62 (t,*J* = 7.2 Hz, 2H), 2.26 (s, 0H), 1.94 (t,*J* = 7.4 Hz, 2H), 1.52 (dt,*J* = 34.3, 7.0 Hz, 5H), 1.39 (t,*J* = 6.9 Hz, 3H), 1.32 - 1.20 (m, 4H). ¹³C NMR (101 MHz, DMSO-*d*₆) δ 169.49, 168.01, 166.20, 157.86, 133.06, 129.12, 128.10, 121.96, 121.65, 121.34, 113.21, 64.50, 41.91, 32.62, 28.56, 27.44, 26.32, 25.41, 14.97.

### Example 15

(Z)-N-hydroxy-7-(5-(4-isopropylbenzylene)-2,4-dioxathiazolidin-3-yl)heptanamide, with a structural formula as below: 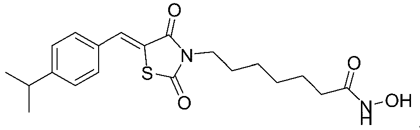 Synthesis method: the fragment (Z)-7-(5-benzylene-2,4-dioxothiazolidin-3-yl)heptylic acid in Step 4 of Example 1 was replaced with (Z)-7-(5-(4-isopropylbenzylene)-2,4-dioxathiazolidin-3-yl)heptylic acid, while other steps and procedures were identical to those in Example 1. White solid, yield: 25%. ¹H NMR (300 MHz, DMSO-*d*₆) δ 10.35 (s, 1H), 8.68 (d,*J* = 1.4 Hz, 1H), 7.90 (s, 1H), 7.57 (d,*J* = 8.1 Hz, 2H), 7.43 (d,*J* = 8.1 Hz, 2H), 3.63 (t,*J* = 7.2 Hz, 2H), 2.94 (h,*J* = 6.9 Hz, 1H), 1.93 (t,*J* = 7.3 Hz, 2H), 1.56 (s, 2H), 1.48 (d,*J* = 6.7 Hz, 0H), 1.23 (t,*J* = 6.3 Hz, 10H). ¹³C NMR (400 MHz, DMSO-*d*₆) δ 170.81, 169.48, 167.85, 166.20, 152.00, 133.36, 131.11, 130.81, 127.87, 120.65, 60.23, 41.94, 33.90, 32.61, 28.55, 27.46, 26.31, 25.41, 23.97, 21.24, 14.56.

### Example 16

(Z)-7-(5-(4-bromo-2-methoxybenzylene)-2,4-dioxathiazolidin-3-yl)-N-hydroxyheptanamide, with a structural formula as below: 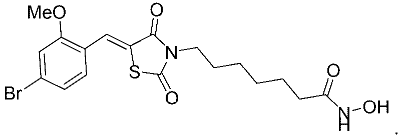 Synthesis method: the fragment (Z)-7-(5-benzylene-2.4-dioxothiazolidin-3-yl)heptylic acid in Step 4 of Example 1 was replaced with (Z)-7-(5-(4-bromo-2-methoxybenzylene)-2.4-dioxathiazolidin-3-yl)heptylic acid, while other steps and procedures were identical to those in Example 1. White solid, yield: 25%. ¹H NMR (400 MHz, DMSO-*d*₆) δ 10.33 (s, 1H), 8.65 (d,*J* = 1.3 Hz, 1H), 7.97 (s, 1H), 7.41 - 7.27 (m, 3H), 3.93 (s, 3H), 3.62 (t,*J* = 7.2 Hz, 2H), 2.04 - 1.89 (m, 2H), 1.51 (dt,*J* = 32.8, 7.1 Hz, 5H), 1.29 - 1.14 (m, 6H), 0.80 (s, 0H). ¹³C NMR (101 MHz, DMSO-*d*₆) δ 169.49, 167.79, 166.12, 158.96, 130.67, 127.04, 125.99, 124.50, 122.64, 121.26, 115.80, 56.89, 41.97, 32.61, 28.55, 27.43, 26.30, 25.40.

### Example 17

(Z)-7-(5-(5-bromo-2-methoxybenzylene)-2,4-dioxathiazolidin-3-yl)-N-hydroxyheptanamide, with a structural formula as below: 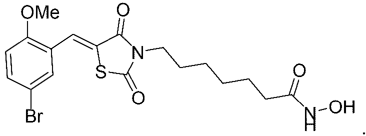 Synthesis method: the fragment (Z)-7-(5-benzylene-2,4-dioxothiazolidin-3-yl)heptylic acid in Step 4 of Example 1 was replaced with (Z)-7-(5-(5-bromo-2-methoxybenzylene)-2,4-dioxathiazolidin-3-yl)heptylic acid, while other steps and procedures were identical to those in Example 1. White solid, yield: 25%. ¹H NMR (400 MHz, DMSO-*d*₆) δ 10.33 (s, 1H), 8.66 (s, 1H), 7.93 (s, 1H), 7.65 (dd,*J* = 8.9, 2.5 Hz, 1H), 7.51 (d,*J* = 2.4 Hz, 1H), 7.14 (d,*J* = 8.9 Hz, 1H), 3.90 (s, 3H), 3.62 (t,*J* = 7.2 Hz, 2H), 2.25 (s, 0H), 1.93 (t,*J* = 7.3 Hz, 2H), 1.52 (dt,*J* = 33.1, 7.1 Hz, 5H), 1.25 (p,*J* = 3.4 Hz, 4H). ¹³C NMR (101 MHz, DMSO-*d*₆) δ 169.49, 167.62, 165.97, 157.50, 135.15, 131.46, 126.90, 124.14, 123.76, 114.76, 112.65, 56.62, 42.00, 32.62, 28.55, 27.42, 26.29, 25.41.

### Example 18

(Z)-7-(5-(3-bromo-2-methoxybenzylene)-2,4-dioxathiazolidin-3-yl)-N-hydroxyheptanamide, with a structural formula as below: 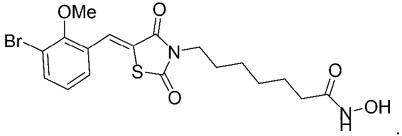 Synthesis method: the fragment (Z)-7-(5-benzylene-2,4-dioxothiazolidin-3-yl)heptylic acid in Step 4 of Example 1 was replaced with (Z)-7-(5-(3-bromo-2-methoxybenzylene)-2,4-dioxathiazolidin-3-yl)heptylic acid, while other steps and procedures were identical to those in Example 1. White solid, yield: 25%. ¹H NMR (400 MHz, DMSO-*d*₆) δ 10.34 (s, 1H), 8.67 (d,*J* = 1.4 Hz, 1H), 7.95 (s, 1H), 7.80 (dd,*J* = 8.0, 1.4 Hz, 1H), 7.53 (dd,*J* = 7.9, 1.4 Hz, 1H), 7.28 (t,*J* = 7.9 Hz, 1H), 3.79 (s, 3H), 3.64 (t,*J* = 7.2 Hz, 2H), 2.26 (t,*J* = 7.3 Hz, 0H), 2.02 - 1.89 (m, 2H), 1.53 (dt,*J* = 38.0, 6.8 Hz, 5H), 1.26 (p,*J* = 3.5 Hz, 4H), 1.18 (t,*J* = 7.1 Hz, 0H). ¹³C NMR (101 MHz, DMSO-*d*₆) δ 169.49, 167.76, 165.99, 156.30, 136.00, 129.06, 129.02, 127.03, 126.91, 124.80, 118.10, 62.43, 60.23, 42.07, 32.61, 28.55, 27.42, 26.31, 25.40, 21.23, 14.55.

### Example 19

(Z)-7-(5-(2-bromo-4-methoxybenzylene)-2,4-dioxathiazolidin-3-yl)-N-hydroxyheptanamide, with a structural formula as below: 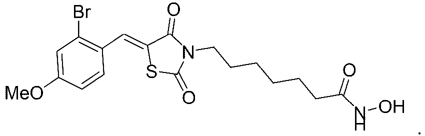 Synthesis method: the fragment (Z)-7-(5-benzylene-2,4-dioxothiazolidin-3-yl)heptylic acid in Step 4 of Example 1 was replaced with (Z)-7-(5-(2-bromo-4-methoxybenzylene)-2,4-dioxathiazolidin-3-yl)heptylic acid, while other steps and procedures were identical to those in Example 1. White solid, yield: 25%. ¹H NMR (400 MHz, DMSO-*d*₆) δ 10.34 (s, 1H), 8.66 (s, 1H), 7.98 (s, 1H), 7.51 (d,*J* = 8.8 Hz, 1H), 7.42 (d,*J* = 2.5 Hz, 1H), 7.16 (dd,*J* = 8.8, 2.6 Hz, 1H), 3.85 (s, 3H), 3.63 (t,*J* = 7.2 Hz, 2H), 2.26 (s, 0H), 1.94 (t,*J* = 7.3 Hz, 2H), 1.58 (q,*J* = 6.9 Hz, 2H), 1.48 (s, 2H), 1.54 - 1.42 (m, 0H), 1.30 - 1.22 (m, 4H). ¹³C NMR (101 MHz, DMSO-*d*₆) δ 169.49, 167.70, 165.96, 161.76, 130.79, 130.59, 127.49, 125.13, 122.46, 119.44, 115.30, 60.23, 56.51, 42.04, 32.61, 28.55, 27.42, 26.31, 25.40, 21.23, 14.55.

### Example 20

(Z)-7-(5-(3-bromo-4-methoxybenzylene)-2,4-dioxathiazolidin-3-yl)-N-hydroxyheptanamide, with a structural formula as below: 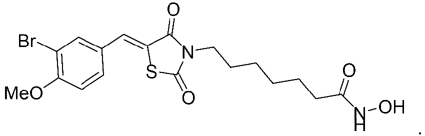 Synthesis method: the fragment (Z)-7-(5-benzylene-2,4-dioxothiazolidin-3-yl)heptylic acid in Step 4 of Example 1 was replaced with (Z)-7-(5-(3-bromo-4-methoxybenzylene)-2,4-dioxathiazolidin-3-yl)heptylic acid, while other steps and procedures were identical to those in Example 1. White solid, yield: 25%. ¹H NMR (400 MHz, DMSO-*d*₆) δ 10.33 (s, 1H), 8.66 (s, 1H), 7.91 - 7.85 (m, 2H), 7.62 (dd,*J* = 8.7, 2.2 Hz, 1H), 7.29 (d,*J* = 8.7 Hz, 1H), 3.93 (s, 3H), 3.63 (t,*J* = 7.2 Hz, 2H), 2.25 (s, 0H), 1.93 (t,*J* = 7.3 Hz, 2H), 1.56 (t,*J* = 6.8 Hz, 2H), 1.47 (q,*J* = 7.1 Hz, 2H), 1.25 (p,*J* = 3.4 Hz, 4H). ¹³C NMR (101 MHz, DMSO-*d*₆) δ 169.49, 167.55, 166.03, 157.46, 135.54, 131.87, 131.21, 127.43, 120.22, 113.74, 111.91, 57.14, 41.97, 32.61, 28.55, 27.45, 26.30, 25.41.

### Example 21

(Z)-7-(5-(3-chloro-2-methoxybenzylene)-2,4-dioxathiazolidin-3-yl)-N-hydroxyheptanamide, with a structural formula as below: 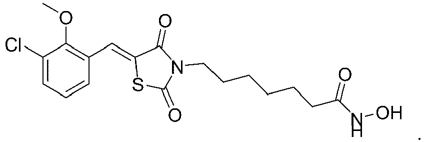 Synthesis method: the fragment (Z)-7-(5-benzylene-2,4-dioxothiazolidin-3-yl)heptylic acid in Step 4 of Example 1 was replaced with (Z)-7-(5-(3-chloro-2-methoxybenzylene)-2,4-dioxathiazolidin-3-yl)heptylic acid, while other steps and procedures were identical to those in Example 1. White solid, yield: 25%. ¹H NMR (300 MHz, DMSO-*d*₆) δ 10.35 (d,*J* = 1.6 Hz, 1H), 8.67 (d,*J* = 1.5 Hz, 1H), 7.96 (s, 1H), 7.66 (dd,*J* = 8.0, 1.5 Hz, 1H), 7.50 (dd,*J* = 7.9, 1.5 Hz, 1H), 7.35 (t,*J* = 7.9 Hz, 1H), 3.82 (s, 3H), 3.64 (t,*J*= 7.2 Hz, 2H), 1.94 (t,*J* = 7.3 Hz, 2H), 1.58 (d,*J* = 7.4 Hz, 2H), 1.59 - 1.42 (m, 1H), 1.48 (s, 2H), 1.26 (td,*J* = 6.9, 3.0 Hz, 5H). ¹³C NMR (101 MHz, DMSO-*d*₆) δ 169.49, 167.63, 165.99, 157.08, 132.27, 128.58, 126.94, 125.10, 123.75, 123.60, 114.29, 56.66, 41.99, 32.61, 28.56, 27.43, 26.30, 25.41.

### Example 22

(Z)-7-(5-(4-chloro-2-methoxybenzylene)-2,4-dioxathiazolidin-3-yl)-N-hydroxyheptanamide, with a structural formula as below: 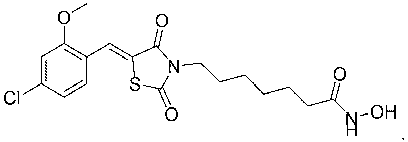 Synthesis method: the fragment (Z)-7-(5-benzylene-2,4-dioxothiazolidin-3-yl)heptylic acid in Step 4 of Example 1 was replaced with (Z)-7-(5-(4-chloro-2-methoxybenzylene)-2,4-dioxathiazolidin-3-yl)heptylic acid, while other steps and procedures were identical to those in Example 1. White solid, yield: 25%. ¹H NMR (400 MHz, DMSO-*d*₆) δ 10.37 (s, 1H), 8.70 (s, 1H), 7.97 (s, 1H), 7.43 (d,*J* = 8.3 Hz, 1H), 7.25 (d,*J* = 1.9 Hz, 1H), 7.17 (dd,*J* = 8.3, 1.9 Hz, 1H), 3.92 (s, 3H), 3.61 (t,*J* = 7.1 Hz, 2H), 1.93 (t,*J* = 7.3 Hz, 2H), 1.77 (t,*J* = 7.3 Hz, 0H), 1.73 - 1.65 (m, 0H), 1.55 (t,*J* = 7.0 Hz, 2H), 1.46 (q,*J* = 6.8 Hz, 2H), 1.35 (q,*J* = 7.0 Hz, 0H), 1.24 (p,*J* = 4.0, 3.6 Hz, 4H). ¹³C NMR (101 MHz, DMSO-*d*₆) δ 169.58, 167.83, 166.13, 159.07, 137.29, 130.54, 126.97, 122.53, 121.54, 120.91, 112.97, 60.25, 56.87, 41.95, 32.60, 28.53, 27.41, 26.28, 25.39, 14.54.

### Example 23

(Z)-7-(5-(5-chloro-2-methoxybenzylene)-2,4-dioxathiazolidin-3-yl)-N-hydroxyheptanamide, with a structural formula as below: 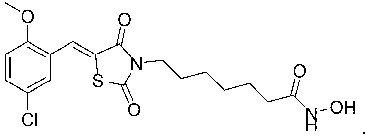 Synthesis method: the fragment (Z)-7-(5-benzylene-2,4-dioxothiazolidin-3-yl)heptylic acid in Step 4 of Example 1 was replaced with (Z)-7-(5-(5-chloro-2-methoxybenzylene)-2,4-dioxathiazolidin-3-yl)heptylic acid, while other steps and procedures were identical to those in Example 1. White solid, yield: 25%. ¹H NMR (400 MHz, DMSO-*d*₆) δ 10.37 - 10.32 (m, 1H), 8.67 (d,*J* = 1.6 Hz, 1H), 7.94 (s, 1H), 7.54 (dd,*J* = 8.9, 2.6 Hz, 1H), 7.39 (d,*J* = 2.6 Hz, 1H), 7.19 (d,*J* = 8.9 Hz, 1H), 3.90 (s, 3H), 3.62 (d,*J* = 14.4 Hz, 1H), 3.35 (s, 1H), 2.25 (s, 0H), 1.93 (t,*J* = 7.4 Hz, 2H), 1.52 (dt,*J* = 32.8, 7.1 Hz, 5H), 1.26 (qd,*J* = 7.7, 5.2, 3.8 Hz, 4H). ¹³C NMR (101 MHz, DMSO-*d*₆) δ 174.78, 171.68, 169.31, 155.78, 155.39, 142.45, 134.54, 129.36, 129.01, 128.98, 124.43, 122.74, 114.01, 65.39, 56.75, 56.73, 42.81, 42.76, 37.23, 33.65, 32.24, 29.30, 29.09, 22.73, 22.03, 18.68, 16.27, 15.64, 13.95.

### Example 24

(Z)-7-(5-(6-chloro-2-methoxybenzylene)-2,4-dioxathiazolidin-3-yl)-N-hydroxyheptanamide, with a structural formula as below: 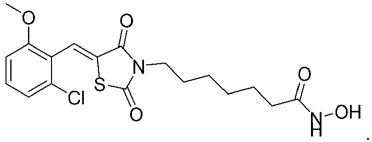 Synthesis method: the fragment (Z)-7-(5-benzylene-2,4-dioxothiazolidin-3-yl)heptylic acid in Step 4 of Example 1 was replaced with (Z)-7-(5-(6-chloro-2-methoxybenzylene)-2,4-dioxathiazolidin-3-yl)heptylic acid, while other steps and procedures were identical to those in Example 1. White solid, yield: 25%. ¹H NMR (300 MHz, DMSO-*d*₆) δ 10.35 (d,*J* = 1.7 Hz, 1H), 8.68 (d,*J* = 1.6 Hz, 1H), 7.92 (s, 1H), 7.49 (t,*J* = 8.3 Hz, 1H), 7.25 - 7.11 (m, 2H), 3.91 (s, 3H), 3.61 (t,*J* = 7.2 Hz, 2H), 2.25 (d,*J* = 7.9 Hz, 0H), 1.94 (t,*J* = 7.3 Hz, 2H), 1.51 (dq,*J* = 21.4, 7.1 Hz, 5H), 1.26 (p,*J* = 3.7 Hz, 4H). ¹³C NMR (101 MHz, DMSO-*d*₆) δ 169.49, 167.74, 166.01, 155.26, 132.99, 129.02, 128.35, 126.67, 126.52, 124.75, 62.38, 42.08, 32.61, 28.55, 27.42, 26.31, 25.40.

### Example 25

(Z)-N-hydroxy-7-(5-(2-methoxy-4-methylbenzylene)-2,4-dioxathiazolidin-3-yl)heptanamide, with a structural formula as below: 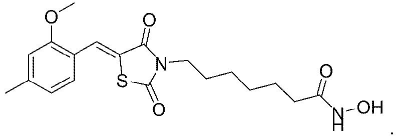 Synthesis method: the fragment (Z)-7-(5-benzylene-2,4-dioxothiazolidin-3-yl)heptylic acid in Step 4 of Example 1 was replaced with (Z)-7-(5-(2-methoxy-4-methylbenzylene)-2,4-dioxathiazolidin-3-yl)heptylic acid, while other steps and procedures were identical to those in Example 1. White solid, yield: 25%. ¹H NMR (400 MHz, DMSO-*d*₆) δ 10.36 (s, 1H), 8.70 (s, 1H), 8.04 (s, 1H), 7.31 (d,*J* = 7.8 Hz, 1H), 7.00 (s, 1H), 6.92 (d,*J* = 7.9 Hz, 1H), 5.75 (s, 0H), 3.88 (s, 3H), 3.61 (t,*J* = 7.2 Hz, 2H), 2.36 (s, 3H), 2.24 (d,*J* = 8.4 Hz, 0H), 1.93 (t,*J* = 7.3 Hz, 2H), 1.50 (dt,*J* = 31.3, 7.0 Hz, 4H), 1.28 - 1.20 (m, 4H). ¹³C NMR (101 MHz, DMSO-*d*₆) δ 169.57, 168.10, 166.31, 158.52, 143.98, 129.20, 128.25, 122.24, 120.36, 119.09, 113.05, 60.25, 56.19, 41.83, 32.61, 28.53, 27.45, 26.29, 25.40, 22.03, 21.22, 14.54.

### Example 26

(Z)-7-(5-(4-fluoro-2-methoxybenzylene)-2,4-dioxathiazolidin-3-yl)-N-hydroxyheptanamide, with a structural formula as below: 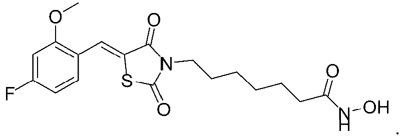 Synthesis method: the fragment (Z)-7-(5-benzylene-2,4-dioxothiazolidin-3-yl)heptylic acid in Step 4 of Example 1 was replaced with (Z)-7-(5-(4-fluoro-2-methoxybenzylene)-2,4-dioxathiazolidin-3-yl)heptylic acid, while other steps and procedures were identical to those in Example 1. White solid, yield: 25%. ¹H NMR (300 MHz, DMSO-*d*₆) δ 10.35 (s, 1H), 8.68 (s, 1H), 7.99 (s, 1H), 7.47 (dd,*J* = 8.7, 6.6 Hz, 1H), 7.11 (dd,*J* = 11.3, 2.4 Hz, 1H), 6.96 (td,*J* = 8.4, 2.4 Hz, 1H), 3.92 (s, 3H), 3.62 (t,*J* = 7.1 Hz, 2H), 1.93 (t,*J* = 7.3 Hz, 2H), 1.51 (dt,*J* = 24.1, 7.0 Hz, 4H), 1.31 - 1.19 (m, 4H). ¹³C NMR (101 MHz, DMSO-*d*₆) δ 169.49, 167.93, 166.19, 160.30, 160.20, 131.19, 131.07, 127.25, 121.53, 118.57, 108.52, 108.30, 101.13, 100.87, 56.93, 41.92, 32.61, 28.55, 27.44, 26.30, 25.40.

### Example 27

(Z)-7-(5-(2,4-dimethoxybenzylene)-2,4-dioxathiazolidin-3-yl)-N-hydroxyheptanamide, with a structural formula as below: 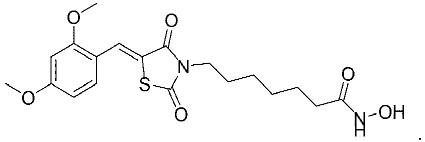 Synthesis method: the fragment (Z)-7-(5-benzylene-2,4-dioxothiazolidin-3-yl)heptylic acid in Step 4 of Example 1 was replaced with (Z)-7-(5-(2,4-dimethoxybenzylene)-2,4-dioxathiazolidin-3-yl)heptylic acid, while other steps and procedures were identical to those in Example 1. White solid, yield: 25%. ¹H NMR (300 MHz, DMSO-*d*₆) δ 10.35 (s, 1H), 8.68 (s, 1H), 8.03 (s, 1H), 7.37 (d,*J* = 8.4 Hz, 1H), 6.71 (d,*J* = 8.5 Hz, 2H), 3.88 (d,*J* = 15.3 Hz, 6H), 3.61 (t,*J* = 7.1 Hz, 2H), 1.93 (t,*J* = 7.3 Hz, 2H), 1.51 (dt,*J* = 22.4, 7.2 Hz, 5H), 1.30 - 1.19 (m, 4H). ¹³C NMR (101 MHz, DMSO-*d*₆) δ 169.49, 168.12, 166.38, 163.74, 160.33, 130.88, 128.14, 118.18, 114.68, 107.06, 99.15, 56.42, 56.15, 41.78, 32.62, 28.56, 27.49, 26.31, 25.41.

### Example 28

(Z)-N-hydroxy-7-(5-(naphth-2-ylmethylene)-2,4-dioxathiazolidin-3-yl)heptanamide, with a structural formula as below: 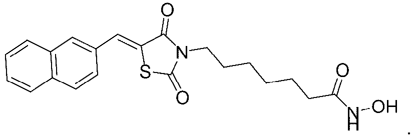 Synthesis method: the fragment (Z)-7-(5-benzylene-2,4-dioxothiazolidin-3-yl)heptylic acid in Step 4 of Example 1 was replaced with (Z)-7-(5-(naphth-2-ylmethylene)-2,4-dioxathiazolidin-3-yl)heptylic acid, while other steps and procedures were identical to those in Example 1. White solid, yield: 25%. ¹H NMR (400 MHz, DMSO-*d*₆) δ 10.35 (s, 1H), 8.67 (s, 1H), 8.33 (s, 0H), 8.22 (s, 1H), 8.06 (d,*J* = 7.6 Hz, 3H), 7.98 (d,*J* = 7.4 Hz, 1H), 7.71 (d,*J* = 8.6 Hz, 1H), 7.62 (p,*J* = 6.6 Hz, 2H), 7.02 (s, 0H), 3.65 (t,*J* = 7.1 Hz, 2H), 2.26 (s, 0H), 1.94 (t,*J* = 7.3 Hz, 2H), 1.76 (s, 0H), 1.63 - 1.54 (m, 2H), 1.48 (q,*J* = 7.0 Hz, 2H), 1.27 (t,*J* = 5.4 Hz, 4H). ¹³C NMR (101 MHz, DMSO-*d*₆) δ 169.52, 167.85, 166.15, 133.80, 133.31, 133.19, 131.56, 131.03, 129.48, 129.22, 128.57, 128.18, 127.65, 126.44, 122.04, 42.00, 32.62, 28.56, 27.45, 26.32, 25.42.

### Example 29

(Z)-7-(2.4-dioxo-5-(quinol-2-ylmethylene)thiazolidin-3-yl)-N-hydroxyheptanamide, with a structural formula as below: 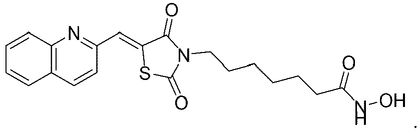 Synthesis method: the fragment (Z)-7-(5-benzylene-2,4-dioxothiazolidin-3-yl)heptylic acid in Step 4 of Example 1 was replaced with (Z)-7-(2,4-dioxo-5-(quinol-2-ylmethylene)thiazolidin-3-yl)heptylic acid, while other steps and procedures were identical to those in Example 1. White solid, yield: 25%. ¹H NMR (300 MHz, DMSO-*d*₆) δ 10.35 (s, 1H), 8.68 (s, 1H), 8.49 (d,*J* = 8.4 Hz, 1H), 8.17 - 8.05 (m, 2H), 7.99 (dd,*J* = 17.3, 8.2 Hz, 2H), 7.85 (t,*J* = 7.7 Hz, 1H), 7.68 (t,*J* = 7.5 Hz, 1H), 3.64 (t,*J* = 7.1 Hz, 2H), 2.26 (s, 0H), 1.94 (t,*J* = 7.3 Hz, 2H), 1.53 (dt,*J* = 28.2, 7.0 Hz, 4H), 1.27 (d,*J* = 5.7 Hz, 4H). ¹³C NMR (101 MHz, DMSO-*d*₆) δ 169.50, 167.50, 165.90, 152.07, 147.80, 136.76, 135.59, 131.92, 129.93, 129.59, 129.23, 128.16, 127.50, 126.98, 124.16, 42.13, 32.62, 28.56, 27.44, 26.32, 25.43.

### Example 30

(Z)-7-(2,4-dioxo-5-(quinol-7-methylene)thiazolidin-3-yl)-N-hydroxyheptanamide, with a structural formula as below: 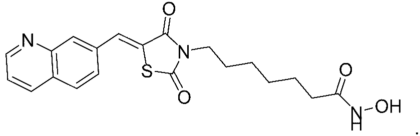 Synthesis method: the fragment (Z)-7-(5-benzylene-2,4-dioxothiazolidin-3-yl)heptylic acid in Step 4 of Example 1 was replaced with (Z)-7-(2,4-dioxo-5-(quinol-7-ylmethylene)thiazolidin-3-yl)heptylic acid, while other steps and procedures were identical to those in Example 1. White solid, yield: 25%. ¹H NMR (400 MHz, DMSO-*d*₆) δ 10.35 (s, 1H), 9.02 - 8.96 (m, 1H), 8.68 (s, 1H), 8.42 (d,*J* = 8.3 Hz, 1H), 8.26 (s, 1H), 8.16 - 8.09 (m, 2H), 7.82 (dd,*J* = 8.5, 1.8 Hz, 1H), 7.62 (dd,*J* = 8.3, 4.2 Hz, 1H), 3.66 (t,*J* = 7.2 Hz, 2H), 2.26 (s, 0H), 1.94 (t,*J* = 7.4 Hz, 2H), 1.60 (q,*J* = 7.0 Hz, 2H), 1.49 (p,*J* = 7.1 Hz, 2H), 1.33 - 1.23 (m, 4H). ¹³C NMR (101 MHz, DMSO-*d*₆) δ 169.46, 167.61, 166.02, 152.34, 147.83, 136.37, 134.50, 132.64, 131.50, 129.79, 129.08, 127.39, 123.65, 123.38, 42.09, 32.62, 28.57, 27.45, 26.32, 25.42.

### Example 31

(Z)-7-(2,4-dioxo-5-(quinol-3-ylmethylene)thiazolidin-3-yl)-N-hydroxyheptanamide, with a structural formula as below: 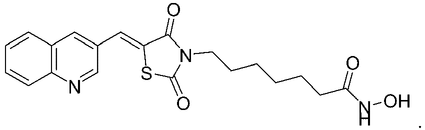 Synthesis method: the fragment (Z)-7-(5-benzylene-2,4-dioxothiazolidin-3-yl)heptylic acid in Step 4 of Example 1 was replaced with (Z)-7-(2,4-dioxo-5-(quinol-3-ylmethylene)thiazolidin-3-yl)heptylic acid, while other steps and procedures were identical to those in Example 1. White solid, yield: 25%. ¹H NMR (400 MHz, DMSO-*d*₆) δ 10.36 (s, 1H), 9.13 (d,*J* = 2.3 Hz, 1H), 9.02 (s, 1H), 8.62 (s, 1H), 8.54 (d,*J* = 2.3 Hz, 1H), 8.20 - 8.09 (m, 2H), 8.06 (d,*J* = 8.4 Hz, 1H), 7.87 (ddd,*J* = 8.4, 6.7, 1.4 Hz, 1H), 7.74 - 7.65 (m, 2H), 7.03 (s, 1H), 3.66 (t,*J* = 7.2 Hz, 2H), 2.26 (t,*J* = 7.4 Hz, 0H), 1.94 (t,*J* = 7.4 Hz, 2H), 1.54 (dp,*J* = 41.0, 7.1 Hz, 5H), 1.36 - 1.19 (m, 5H). ¹³C NMR (101 MHz, DMSO-*d*₆) δ 169.49, 168.01, 166.01, 158.03, 134.85, 133.09, 127.64, 127.35, 122.37, 120.07, 111.32, 56.27, 41.92, 32.62, 28.54, 27.48, 26.36, 25.42.

### Example 32

(Z)-7-(2,4-dioxy-5-(quinol-6-ylmethylene)thiazolidin-3-yl)-N-hydroxyheptanamide, with a structural formula as below: 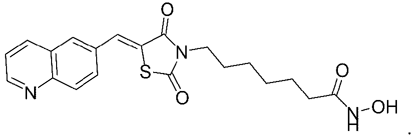 Synthesis method: the fragment (Z)-7-(5-benzylene-2,4-dioxothiazolidin-3-yl)heptylic acid in Step 4 of Example 1 was replaced with (Z)-7-(2,4-dioxo-5-(quinol-6-ylmethylene)thiazolidin-3-yl)heptylic acid, while other steps and procedures were identical to those in Example 1. White solid, yield: 25%. ¹H NMR (400 MHz, DMSO-*d*₆) δ 10.35 (s, 1H), 8.98 (d,*J* = 4.1 Hz, 1H), 8.68 (s, 1H), 8.50 (d,*J* = 8.3 Hz, 1H), 8.26 (s, 1H), 8.17 - 8.06 (m, 2H), 8.00 - 7.92 (m, 1H), 7.62 (dd, *J* = 8.3, 4.2 Hz, 1H), 3.66 (t,*J* = 7.2 Hz, 2H), 2.27 (d,*J* = 8.6 Hz, 0H), 1.94 (t,*J* = 7.3 Hz, 2H), 1.54 (dp,*J* = 40.8, 7.1 Hz, 5H), 1.31 - 1.13 (m, 4H). ¹³C NMR (101 MHz, DMSO-*d*₆) δ 169.46, 167.75, 166.08, 152.77, 148.23, 137.32, 132.47, 131.61, 131.26, 130.51, 130.39, 128.33, 123.12, 122.98, 42.06, 32.61, 28.56, 27.44, 26.32, 25.42.

### Example 33

(Z)-7-(5-((2,3-dihydrobenzofuran-5-yl)methylene)-2,4-dioxathiazolidin-3-yl)-N-hydroxyheptanamide, with a structural formula as below: 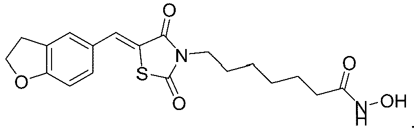 Synthesis method: the fragment (Z)-7-(5-benzylene-2,4-dioxothiazolidin-3-yl)heptylic acid in Step 4 of Example 1 was replaced with (Z)-7-(2,4-dioxo-5-(quinol-6-ylmethylene)thiazolidin-3-yl)heptylic acid, while other steps and procedures were identical to those in Example 1. White solid, yield: 25%. ¹H NMR (300 MHz, DMSO-*d*₆) δ 10.35 (d,*J* = 1.5 Hz, 1H), 8.68 (d,*J* = 1.6 Hz, 1H), 7.86 (s, 1H), 7.54 - 7.37 (m, 2H), 6.94 (d,*J* = 8.4 Hz, 1H), 4.63 (t,*J* = 8.7 Hz, 2H), 3.62 (t,*J* = 7.1 Hz, 2H), 3.26 (t,*J* = 8.7 Hz, 2H), 1.93 (t,*J* = 7.3 Hz, 2H), 1.59 - 1.42 (m, 5H), 1.26 (q,*J* = 4.1, 3.6 Hz, 5H). ¹³C NMR (101 MHz, DMSO-*d*₆) δ 169.49, 167.88, 166.29, 162.55, 133.90, 132.42, 129.71, 127.55, 125.98, 117.56, 110.41, 72.51, 41.84, 32.61, 29.04, 28.55, 27.48, 26.31, 25.41.

### Example 34

(Z)-7-(5-(benzo[d][1,3] dioxazol5-ylmethylene)-2,4-dioxathiazolin-3-yl)-N-hydroxyheptanamide, with a structural formula as below: 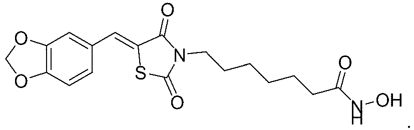 Synthesis method: the fragment (Z)-7-(5-benzylene-2,4-dioxothiazolidin-3-yl)heptylic acid in Step 4 of Example 1 was replaced with (Z)-7-(5-(benzo[d][1,3]dihydroxy-5-ylmethylene)-2,4-dioxathiazolidin-3-yl)heptylic acid, while other steps and procedures were identical to those in Example 1. White solid, yield: 25%. ¹H NMR (400 MHz, DMSO-*d*₆) δ 10.35 (s, 1H), 8.68 (s, 1H), 8.48 (d,*J* = 8.4 Hz, 1H), 8.15 - 8.05 (m, 2H), 7.99 (dd,*J* = 24.0, 8.2 Hz, 2H), 7.84 (t,*J* = 7.6 Hz, 1H), 7.68 (t,*J* = 7.5 Hz, 1H), 3.64 (t,*J* = 7.2 Hz, 2H), 2.26 (s, 0H), 1.94 (t,*J* = 7.4 Hz, 2H), 1.58 (q,*J* = 7.0 Hz, 2H), 1.48 (t,*J* = 7.2 Hz, 2H), 1.27 (dd,*J* = 6.9, 3.4 Hz, 4H). ¹³C NMR (101 MHz, DMSO-*d*₆) δ 172.01, 169.50, 166.30, 152.24, 147.34, 137.88, 131.18, 128.89, 128.74, 128.51, 128.33, 128.06, 127.50, 124.95, 41.21, 32.62, 28.57, 27.55, 26.34, 25.44.

### Example 35

(Z)-7-(2,4-dioxy-5-(pyridin-3-ylmethylene)thiazolidin-3-yl)-N-hydroxyheptanamide, with a structural formula as below: 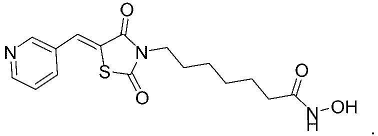 Synthesis method: the fragment (Z)-7-(5-benzylene-2,4-dioxothiazolidin-3-yl)heptylic acid in Step 4 of Example 1 was replaced with (Z)-7-(2,4-dioxo-5-(pyridin-3-ylmethylene)thiazolidin-3-yl)heptylic acid, while other steps and procedures were identical to those in Example 1. White solid, yield: 25%. ¹H NMR (400 MHz, DMSO-*d*₆) δ 10.33 (s, 1H), 8.86 (d,*J* = 2.3 Hz, 1H), 8.68 - 8.61 (m, 2H), 8.03 - 7.95 (m, 2H), 7.58 (dd,*J* = 8.0, 4.8 Hz, 1H), 3.65 (t,*J* = 7.2 Hz, 2H), 2.28 - 2.21 (m, 0H), 1.93 (t,*J* = 7.3 Hz, 2H), 1.52 (dq,*J* = 38.6, 6.9 Hz, 5H), 1.27 (qd,*J* = 7.5, 5.0, 3.5 Hz, 4H). ¹³C NMR (101 MHz, DMSO-*d*₆) δ 169.49, 167.41, 165.85, 151.81, 151.17, 136.56, 130.00, 129.62, 124.70, 124.12, 42.12, 32.61, 28.55, 27.41, 26.29, 25.41.

### Example 36

(Z)-7-(2,4-dioxo-5-(quinol-8-methylene)thiazolidin-3-yl)-N-hydroxyheptanamide, with a structural formula as below: 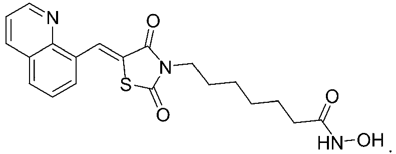 Synthesis method: the fragment (Z)-7-(5-benzylene-2,4-dioxothiazolidin-3-yl)heptylic acid in Step 4 of Example 1 was replaced with (Z)-7-(2,4-dioxo-5-(quinol-8-methylene)thiazolidin-3-yl)heptylic acid, while other steps and procedures were identical to those in Example 1. White solid, yield: 25%. ¹H NMR (400 MHz, DMSO-*d*₆) δ 10.34 (d,*J* = 1.5 Hz, 1H), 9.08 - 8.97 (m, 2H), 8.66 (d,*J* = 1.7 Hz, 1H), 8.49 (dd,*J* = 8.3, 1.8 Hz, 1H), 8.16 (dd,*J* = 8.2, 1.3 Hz, 1H), 7.95 (dd,*J* = 7.4, 1.2 Hz, 1H), 7.78 (t,*J* = 7.8 Hz, 1H), 7.69 (dd,*J* = 8.3, 4.2 Hz, 1H), 3.66 (t,*J* = 7.2 Hz, 2H), 2.27 (s, 0H), 1.94 (t,*J* = 7.4 Hz, 2H), 1.53 (dq,*J* = 41.5, 7.2, 6.8 Hz, 5H), 1.28 (p,*J* = 3.6 Hz, 4H). ¹³C NMR (101 MHz, DMSO-*d*₆) δ 169.50, 168.38, 166.30, 151.00, 145.84, 137.45, 131.64, 131.26, 130.64, 129.59, 128.63, 127.11, 123.56, 123.00, 60.22, 41.92, 32.63, 28.58, 27.49, 26.34, 25.43, 14.55.

### Example 37

(Z)-7-(2,4-dioxo-5-(thien-2-ylmethylene)thiazolidin-3-yl)-N-hydroxyheptanamide, with a structural formula as below: 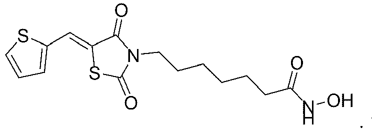 Synthesis method: the fragment (Z)-7-(5-benzylene-2,4-dioxothiazolidin-3-yl)heptylic acid in Step 4 of Example 1 was replaced with (Z)-7-(2,4-dioxo-5-(thien-2-ylmethylene)thiazolidin-3-yl)heptylic acid, while other steps and procedures were identical to those in Example 1. White solid, yield: 25%. ¹H NMR (400 MHz, DMSO-*d*₆) δ 10.37 (s, 1H), 8.70 (s, 1H), 8.18 (s, 1H), 8.03 (d,*J* = 4.9 Hz, 1H), 7.71 (d,*J* = 3.6 Hz, 1H), 7.30 (dd,*J* = 5.0, 3.7 Hz, 1H), 3.61 (t,*J* = 7.2 Hz, 2H), 2.25 (t,*J* = 7.5 Hz, 0H), 1.93 (t,*J* = 7.3 Hz, 2H), 1.51 (dt,*J* = 33.6, 7.0 Hz, 4H), 1.30 - 1.20 (m, 4H). ¹³C NMR (101 MHz, DMSO-*d*₆) δ 169.58, 167.11, 165.87, 137.56, 135.46, 133.93, 129.49, 126.68, 119.09, 60.25, 42.08, 32.60, 28.53, 27.45, 26.28, 25.40, 21.22, 14.54.

### Example 38

(Z)-7-(5-(furan-2-ylmethylene)-2,4-dioxathiazolidin-3-yl)-N-hydroxyheptanamide, with a structural formula as below: 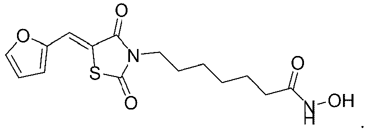 Synthesis method: the fragment (Z)-7-(5-benzylene-2,4-dioxothiazolidin-3-yl)heptylic acid in Step 4 of Example 1 was replaced with (Z)-7-(2,4-dioxo-5-(furan-2-ylmethylene)thiazolidin-3-yl)heptylic acid, while other steps and procedures were identical to those in Example 1. White solid, yield: 25%. ¹H NMR (400 MHz, DMSO-*d*₆) δ 10.36 (s, 1H), 8.70 (s, 1H), 8.07 (d,*J* = 1.7 Hz, 1H), 7.74 (s, 1H), 7.14 (d,*J* = 3.5 Hz, 1H), 6.76 (dd,*J* = 3.5, 1.8 Hz, 1H), 3.60 (t,*J* = 7.2 Hz, 2H), 1.93 (t,*J* = 7.4 Hz, 2H), 1.50 (dt,*J* = 30.9, 7.2 Hz, 4H), 1.28 - 1.19 (m, 4H). ¹³C NMR (101 MHz, DMSO-*d*₆) δ 169.58, 168.53, 165.92, 149.68, 148.20, 119.84, 119.62, 118.43, 114.09, 60.25, 41.78, 32.59, 28.52, 27.46, 26.27, 25.40, 21.22, 14.54.

### Example 39

(Z)-N-hydroxy-7-(5-(4-methoxybenzylene)-4-oxo-2-thiothiazolidin-3-yl)heptanamide, with a structural formula as below: 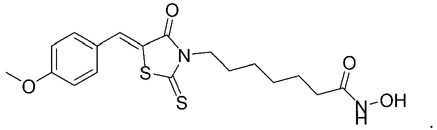 Synthesis method: the fragment (Z)-7-(5-benzylene-2,4-dioxothiazolidin-3-yl)heptylic acid in Step 4 of Example 1 was replaced with (Z)-7-(5-(4-methoxybenzylene)-4-oxo-2-thiothiazolidin-3-yl)heptylic acid, while other steps and procedures were identical to those in Example 1. White solid, yield: 25%. ¹H NMR (400 MHz, DMSO-*d*₆) δ 10.37 (s, 1H), 8.70 (s, 1H), 7.84 (s, 0H), 7.76 (s, 1H), 7.60 (d,*J* = 8.4 Hz, 2H), 7.18 (s, 0H), 7.12 (d,*J* = 8.5 Hz, 2H), 4.00 (t,*J* = 7.4 Hz, 2H), 3.84 (s, 3H), 1.93 (t,*J* = 7.4 Hz, 2H), 1.61 (p,*J* = 7.2 Hz, 2H), 1.47 (t,*J* = 7.2 Hz, 2H), 1.30 - 1.22 (m, 4H). ¹³C NMR (101 MHz, DMSO-*d*₆) δ 193.68, 169.58, 167.49, 162.00, 133.67, 133.41, 125.98, 119.47, 115.63, 60.25, 56.06, 44.60, 32.59, 28.57, 26.71, 26.36, 25.40, 14.54.

### Example 40

(Z)-7-(5-(4-chlorobenzylene)-4-oxo-2-thiathiazolidin-3-yl)-N-hydroxyheptanamide, with a structural formula as below: 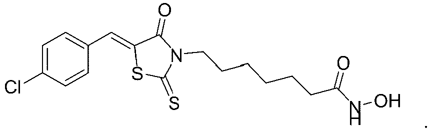 Synthesis method: the fragment (Z)-7-(5-benzylene-2,4-dioxothiazolidin-3-yl)heptylic acid in Step 4 of Example 1 was replaced with (Z)-7-(5-(4-chlorobenzylene)-4-oxo-2-thiothiazolidin-3-yl)heptylic acid, while other steps and procedures were identical to those in Example 1. White solid, yield: 25%. ¹H NMR (400 MHz, DMSO-*d*₆) δ 10.37 (s, 1H), 8.70 (s, 1H), 7.79 (s, 1H), 7.63 (q,*J* = 8.4 Hz, 4H), 4.00 (t,*J* = 7.4 Hz, 2H), 2.25 (s, 0H), 1.93 (t,*J* = 7.3 Hz, 2H), 1.61 (p,*J* = 7.9, 7.4 Hz, 2H), 1.47 (t,*J* = 7.2 Hz, 2H), 1.35 - 1.19 (m, 5H). ¹³C NMR (101 MHz, DMSO-*d*₆) δ 193.64, 169.57, 167.36, 136.06, 132.69, 132.32, 131.93, 130.04, 123.62, 60.25, 44.70, 32.59, 28.57, 26.69, 26.35, 25.39, 21.22, 14.54.

### Example 41

(Z)-N-hydroxy-7-(5-(4-methylbenzylene)-4-oxo-2-thiothiazolidin-3-yl)heptanamide, with a structural formula as below: 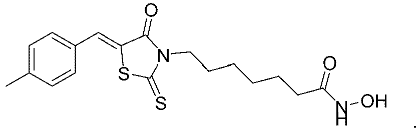 Synthesis method: the fragment (Z)-7-(5-benzylene-2,4-dioxothiazolidin-3-yl)heptylic acid in Step 4 of Example 1 was replaced with (Z)-7-(5-(4-methylbenzylene)-4-oxo-2-thiothiazolidin-3-yl)heptylic acid, while other steps and procedures were identical to those in Example 1. White solid, yield: 25%. ¹H NMR (300 MHz, DMSO-*d*₆) δ 10.35 (s, 1H), 8.68 (s, 1H), 7.78 (s, 1H), 7.54 (d,*J* = 7.8 Hz, 2H), 7.38 (d,*J* = 7.8 Hz, 2H), 4.01 (t,*J* = 7.4 Hz, 2H), 2.37 (s, 3H), 2.25 (s, 0H), 1.93 (t,*J* = 7.3 Hz, 2H), 1.61 (q,*J* = 7.3 Hz, 2H), 1.48 (p,*J* = 7.4 Hz, 2H), 1.28 (s, 5H).

### Example 42

(Z)-7-(5-(4-ethylbenzylene)-4-oxo-2-thiothiazolidin-3-yl)-N-hydroxyheptanamide, with a structural formula as below: 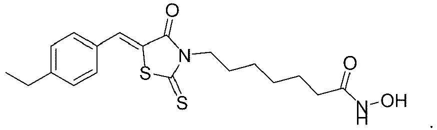 Synthesis method: the fragment (Z)-7-(5-benzylene-2,4-dioxothiazolidin-3-yl)heptylic acid in Step 4 of Example 1 was replaced with (Z)-7-(5-(4-ethylbenzylene)-4-oxo-2-thiothiazolidin-3-yl)heptylic acid, while other steps and procedures were identical to those in Example 1. White solid, yield: 25%. ¹H NMR (300 MHz, DMSO-*d*₆) δ 10.35 (d,*J* = 1.7 Hz, 1H), 8.68 (d,*J* = 1.7 Hz, 1H), 7.79 (s, 1H), 7.57 (d,*J* = 8.2 Hz, 2H), 7.41 (d,*J* = 8.0 Hz, 2H), 4.08 - 3.95 (m, 2H), 2.67 (q,*J* = 7.6 Hz, 2H), 1.93 (t,*J* = 7.3 Hz, 2H), 1.62 (s, 2H), 1.54 - 1.42 (m, 2H), 1.32 - 1.14 (m, 8H). ¹³C NMR (101 MHz, DMSO-*d*₆) δ 193.88, 169.48, 167.46, 148.02, 133.56, 131.35, 131.01, 129.47, 121.66, 44.65, 32.60, 28.63, 28.59, 26.72, 26.38, 25.41, 15.61.

### Example 43

(Z)-N-(2-amino-4-fluorophenyl)-4-((2,4-dioxy-5-(pyridin-3-ylmethylene)thiazolidin-3-yl)methyl)benzamide, with a structural formula as below: 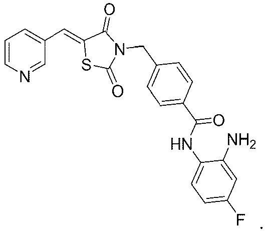 Thiazolidinone (5 mmol, 585.6 mg), acetic acid (10mL) and 3-pyridylaldehyde (5 mmol, 530.6 mg) were sequentially added to a 100 mL round-bottom flask and refluxed with stirring at 120°C to allow a reaction to proceed for 8 h. A reaction mixture was cooled to room temperature for precipitation of solids, and then subjected to suction filtration; a filter cake was dried to obtain 820 mg of white solid; the white solid was added to a round-bottom flask; 10 mL of DMF was added as a solvent, and 1.5 eq. of potassium carbonate (6 mmol) was further added; a resulting mixture was pre-stirred for 30 min in an ice bath; the ice bath was removed, allowing the temperature to rise to room temperature during reaction; 2 eq. of methyl 4-bromomethyl benzoate was added; the mixture was stirred to allow a reaction to proceed for 8 h; then, the reaction mixture was extracted with water and ethyl acetate; and organic layers were collected, concentrated, and eluted and separated by column chromatography using a mixed solvent (petroleum ether: ethyl acetate = 3:1 by volume) as an eluent to obtain a milk-white solid. 3 mL of glacial acetic acid and 0.5 mL of concentrated hydrochloric acid (10 M) were added to the milk-white solid (2 mmol); a resulting mixture was heated to 90°C and stirred for reaction, with a reaction process monitored by TLC until full consumption of the starting materials; the reaction mixture was cooled, followed by slow dropwise addition of a saturated aqueous sodium chloride solution for precipitation of solids; the reaction mixture was filtered; and a filter cake was washed three times with water and dried to obtain a white solid. The white solid was dissolved in 5 mL of DMF; DIPEA (0.15 mmol, 20 mg), HATU (0.15 mmol, 57 mg) and para-fluoro-o-phenylenediamine (40 mg, 0.3 mmol) were added; a resulting mixture was stirred for 6 h at room temperature; a reaction mixture was concentrated and eluted by column chromatography using a mixed solvent (dichloromethane: methanol = 25:1 by volume) as an eluent to obtain (Z)-N-(2-amino-4-fluorophenyl)-4-((2,4-dioxy-5-(pyridin-3-ylmethylene)thiazolidin-3-yl)methyl)benzamide as a white solid, with the yield of 32%. ¹H NMR (400 MHz, DMSO-*d₆*) δ 9.59 (s, 1H), 8.89 (d,*J* = 2.4 Hz, 1H), 8.66 (dd,*J* = 4.8, 1.5 Hz, 1H), 8.21-7.87 (m, 4H), 7.59 (dd,*J* = 8.0, 4.8 Hz, 1H), 7.45 (dd,*J* = 8.5, 1.9Hz, 2H), 7.11 (dd,*J* = 8.7, 6.4 Hz, 1H), 6.54 (dd,*J* = 11.2, 2.9Hz, 1H), 6.35 (td,*J* = 8.5, 2.9 Hz, 1H), 5.23 (s, 2H), 4.94 (s, 2H).
The beneficial effects and uses of the compounds represented by the formulae in the present invention are illustrated by the following experiments.

HDAC1 inhibition assays were conducted for the inhibitors of the present invention:
the HDAC1 inhibitory activity of the compounds of the present invention was tested using the ADP-Glo Luminescent Kinase Assay. The specific experimental results were described by referring to the following references: Eur J. Med. Chem., 2020, 204, 112637; and Eur J. Med. Chem., 2022, 229, 114055. Reagents used in the kinase reaction were as follows: HEPES (50 mM) pH 7.5 with NaCl (100 mM), EGTA (1.0 mM), MgCl₂(3.0 mM), DTT (2.0 mM) and CHAPS (0.03%). During the reaction, 50 µM PIP2 and 25 µM ATP were added to 10 mL of each test compound at varying concentrations (0.05 nM to 1.0 µM). A reaction mixture was incubated at room temperature for 1 h, and then, 10 µL of an ADP-Glo reagent was added to terminate an enzymatic reaction. Data were collected using Envision software, and the IC₅₀ values of the compounds were analyzed and fitted using Graphpad Prism 5.

**Table 1: HDAC1 inhibitory activity (IC₅₀, nM) of compounds of examples**

| Example 1 | Example 2 | Example 3 | Example 4 | Example 5 | Example 6 | Example 7 |
|---|---|---|---|---|---|---|
| 3.2 | 6.1 | 2.5 | 2.1 | 4.0 | 5.0 | 6.7 |

| Example 8 | Example 9 | Example 10 | Example 11 | Example 12 | Example 13 | Example 14 |
|---|---|---|---|---|---|---|
| 3.3 | 2.2 | 3.5 | 3.6 | 4.8 | 2.6 | 3.9 |

| Example 16 | Example 17 | Example 18 | Example 19 | Example 20 | Example 21 | Example 22 |
|---|---|---|---|---|---|---|
| 1.9 | 2.6 | 2.8 | 4.1 | 2.4 | 3.2 | 4.4 |

| Example 23 | Example 24 | Example 25 | Example 26 | Example 27 | Example 28 | Example 29 |
|---|---|---|---|---|---|---|
| 2.3 | 3.4 | 3.6 | 2.4 | 3.0 | 3.4 | 2.5 |

| Example 30 | Example 31 | Example 32 | Example 33 | Example 34 | Example 35 | Example 36 |
|---|---|---|---|---|---|---|
| 5.6 | 4.2 | 1.8 | 1.9 | 2.3 | 10 | 1.3 |

| Example 37 | Example 38 | Example 40 | Example 41 | Example 42 | Vorinostat | Tucidinostat |
|---|---|---|---|---|---|---|
| 3.1 | 4.2 | 11 | 8.5 | 31 | 15.0 | 122.1 |

The HDAC1 inhibitory activity of all the examples is shown in Table 1. As can be seen from the table, most of the compounds exhibit a nanomolar-level inhibitory activity against HDAC1, showing significant superiority to the positive control Vorinostat. In particular, Examples 16, 32, 33 and 36 exhibit significantly superior HDAC1 inhibitory activity to the positive control Vorinostat. This indicates that the compounds in the examples of the present invention are efficient HDAC1 inhibitors.

MTT assay was used for measurement in this experiment, with specific steps as follows. Tumor cells in the logarithmic growth phase were digested and dispersed with EDTA-trypsin; the EDTA-trypsin was then washed away; a single-cell suspension was prepared using a culture medium and then adjusted to the concentration of 2000 cells per 100 µL of the culture medium; the suspension was added to a 96-well plate at 100 µL per well; and the cells were incubated in an incubator for 24 h for the cells to adhere to the wall and grow. A blank group, a negative control group (DMSO) and a treatment group were established. Each well was supplemented with 100 µL of the culture medium to achieve a corresponding concentration, followed by incubation of the cells in the incubator for 96 h. 10 µL of an MTT solution (5 mg/mL) was then added to each well under light-proof conditions; the cells were incubated in the incubator for another 4 h; the supernatant in each well was carefully removed with a pipette; and 150 µL of DMSO was added and shaken for through mixing. The absorbance of each well was measured at 570 nm using a Molecular Devices ID5 multifunctional microplate reader, and the results were calculated according to the formula below and then fitted using Graphad Grism to obtain IC₅₀: $inhibition rate \left(%\right) = \left[1-\left({OD}_{treatment}-{OD}_{blank}\right)/\left({OD}_{positive control}-{OD}_{blank}\right)\right] \times 100 % .$

**Table 2: Anti-proliferative activity (IC₅₀, µM) of compounds of examples against tumor cell lines**

| Example | Example 1 | Example 2 | Example 3 | Example 4 | Example 5 | Example 6 | Example 7 | Example 8 |
|---|---|---|---|---|---|---|---|---|
| A549 | 3.10 | 5.36 | 6.44 | 4.71 | 4.31 | 3.67 | 1.61 | 3.62 |
| DU145 | 0.51 | 1.23 | 0.86 | 0.84 | 1.22 | 1.29 | 1.05 | 1.64 |

| Example | Example 9 | Example 10 | Example 11 | Example 12 | Example 13 | Example 14 | Example 15 | Example 16 |
|---|---|---|---|---|---|---|---|---|
| A549 | 1.34 | 1.37 | 1.60 | 5.37 | 1.52 | 3.36 | 5.64 | 4.00 |
| DU145 | 0.50 | 0.29 | 1.09 | 4.19 | 0.29 | 0.50 | 3.81 | 1.05 |

| Example | Example 17 | Example 18 | Example 19 | Example 20 | Example 21 | Example 22 | Example 23 | Example 24 |
|---|---|---|---|---|---|---|---|---|
| A549 | 1.50 | 1.56 | 2.28 | 2.51 | 3.78 | 5.96 | 2.82 | 7.52 |
| DU145 | 3.57 | 0.74 | 0.97 | 0.53 | 2.00 | 1.97 | 1.27 | 3.26 |

| Example | Example 25 | Example 26 | Example 27 | Example 28 | Example 29 | Example 30 | Example 31 | Example 32 |
|---|---|---|---|---|---|---|---|---|
| A549 | 2.00 | 2.71 | 0.91 | 2.44 | 0.55 | 1.07 | 3.83 | 10.08 |
| DU145 | 1.15 | 0.93 | 0.74 | 1.39 | 6.56 | <0.10 | 2.10 | 2.40 |

| Example | Example 33 | Example 34 | Example 35 | Example 36 | Example 37 | Example 38 | Example 39 | Example 40 |
|---|---|---|---|---|---|---|---|---|
| A549 | 2.08 | 2.47 | 10.91 | 2.69 | 2.32 | 1.26 | 3.09 | 5.65 |
| DU145 | 9.18 | 1.06 | 3.18 | 0.22 | 0.33 | 0.16 | 2.46 | 3.62 |

| Example | Example 41 | Example 42 | Vorinostat | Tucidinostat | | | | |
|---|---|---|---|---|---|---|---|---|
| A549 | 2.98 | 2.70 | 1.62 | 9.85 | | | | |
| DU145 | 3.31 | 3.64 | 0.64 | 1.62 | | | | |

Table 2 shows the anti-proliferative activity of the compounds of examples against two types of tumor cell lines. As can be seen from the table, the compounds of the examples exhibit a micromolar-level anti-proliferative activity against the two types of tumor cell lines, and in particular, most of the compounds of the examples show high sensitivity towards the human prostate cancer cell line DU145. Here, the 10 compounds including that of Example 1 exhibit the inhibitory activity of less than 0.64 µM against DU145 in Table 2, while the compounds of Examples 27 and 29 exhibit the submicromolar-level inhibitory activity against the human non-small cell lung cancer cell line A549 in Table 2. These compounds are superior to the positive control Vorinostat. In particular, the compound of Example 30 exhibits the nanomolar-level inhibitory activity of less than 100 nM against DU145 in Table 2, which is significantly superior to that of the positive control Vorinostat. As can be seen from this, the compounds in the examples of the present invention can be potentially applied to the research of anti-tumor drugs described above for clinical treatment.

In summary, the compounds protected by the present invention are HDAC1 inhibitors, which are highly effective in inhibiting HDAC1 and the proliferation of various solid and hematological tumor cells and have high sensitivity towards prostate cancer, showing potential for the clinical treatment of the prostate cancer.

In addition to the implementations described above, other embodiments are also possible for the present invention. Any technical solutions derived by equivalent substitution or transformation shall fall within the scope of protection of the present invention.

## Claims

1. A thiazolidinedione-based HDAC inhibitor, comprising a substituted thiazolidinedione compound of Formula V, or a stereisomer, hydrate or pharmaceutically acceptable salt thereof: 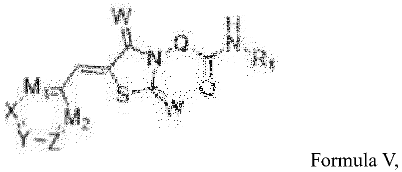
wherein W is selected from the group consisting of an oxygen atom and a sulfur atom; X, Y, Z, M₁ and M₂ each independently represent a carbon or nitrogen atom, when X, Y, Z, M₁ or M₂ is a carbon atom, each is independently and optionally substituted by R₂, and R₂ can be hydrogen, alkyl, cyano, halogen, haloalkyl, hydroxy, mercapto, alkoxy, alkylthio, alkoxyalkyl, arylalkyl, aryl or Het;
Q is selected from the group consisting of a saturated or unsaturated straight or branched hydrocarbon group having a length of 1 to 9 carbon atoms, aryl and Het;
R₁ is selected from the group consisting of hydroxy, and 2-aminophenyl optionally substituted by one or more R₃;
R₃ can be hydrogen, alkyl, cyano, halogen, haloalkyl, hydroxy, mercapto, alkoxy, alkylthio, alkoxyalkyl, arylalkyl, aryl or Het;
the alkyl is a straight or branched saturated hydrocarbon group having 1 to 9 carbon atoms, or a cyclic saturated hydrocarbon group having 3 to 6 carbon atoms, or a cyclic saturated hydrocarbon group having 3 to 6 carbon atoms and linked to a straight or branched saturated hydrocarbon group having 1 to 6 carbon atoms;
the alkoxy is a straight or branched saturated hydrocarbon group having 1 to 9 carbon atoms, or a cyclic saturated hydrocarbon group having 3 to 9 carbon atoms, or a cyclic saturated hydrocarbon group having 3 to 6 carbon atoms and linked to a straight or branched saturated hydrocarbon group having 1 to 9 carbon atoms, wherein each carbon atom is optionally substituted by an oxygen atom;
the alkylamino is a straight or branched saturated hydrocarbon group having 1 to 9 carbon atoms, or a cyclic saturated hydrocarbon group having 3 to 6 carbon atoms, or a cyclic saturated hydrocarbon group having 3 to 6 carbon atoms and linked to a straight or branched saturated hydrocarbon group having 1 to 9 carbon atoms, wherein each carbon atom is optionally substituted by an NH atom;
the alkoxyalkyl represents linking the alkoxy defined as above to alkyl;
alkenyl or alkynyl is a straight or branched unsaturated hydrocarbon group having 1 to 9 carbon atoms and a double or triple bond;
the aryl is a carboatomic ring selected from the group consisting of phenyl, naphthyl, acenaphthenyl and tetrahydronaphthyl, each of which is optionally substituted by 1, 2 or 3 substituents, with each substituent independently selected from the group consisting of hydrogen, alkyl, cyano, halogen, haloalkyl, hydroxy, mercapto, alkoxy, alkylthio, alkoxyalkyl, arylalkyl, diarylalkyl, aryl and Het;
the arylalkyl or diarylalkyl represents linking the aryl defined as above to alkyl;
the Het is a monoheterocyclic ring selected from the group consisting of pyrrolyl, pyrazolyl, imidazolyl, furyl, thienyl, oxazolyl, isoxazolyl, thiazolyl, isothiazolyl, pyridinyl, pyrimidinyl, pyrazinyl and pyridazinyl, or is a biheterocyclic ring selected from the group consisting of quinolyl, quinoxalinyl, indolyl, benzoimidazolyl, benzoxazolyl, benzoisoxazolyl, benzothiazolyl, benzoisothiazolyl, benzofuryl, benzothienyl, 2,3-dihydrobenzo[1,4]dioxin and benzo[1,3]dioxol, or is selected from the group consisting of a monocyclic saturated hydrocarbon group having 3 to 6 carbon atoms, and a bicyclic saturated hydrocarbon group having 6 to 12 carbon atoms, wherein a carbon atom on a ring is independently and optionally substituted by 1 to 4 O, S, N or NH; each monocyclic or bicyclic ring is optionally substituted by 1, 2 or 3 substituents, with each substituent independently selected from the group consisting of halogen, haloalkyl, hydroxy, alkyl and alkoxy;
the halogen is a substituent selected from the group consisting of fluorine, chlorine, bromine and iodine; and the haloalkyl is a straight or branched saturated hydrocarbon group having 1 to 9 carbon atoms, or a cyclic saturated hydrocarbon group having 3 to 6 carbon atoms, or a cyclic saturated hydrocarbon group having 3 to 6 carbon atoms and linked to a straight or branched saturated alkyl group having 1 to 6 carbon atoms, wherein one or more carbon atoms are substituted by one or more halogen atoms.

2. The thiazolidinedione-based HDAC inhibitor according to claim 1, wherein
in the Formula V, W is selected from the group consisting of an oxygen atom and a sulfur atom; X, Y, Z, M₁ and M₂ each independently represent a carbon or nitrogen atom, when X, Y, Z, M₁ or M₂ is a carbon atom, each is independently and optionally substituted by R₂, and R₂ is hydrogen, alkyl, cyano, halogen, haloalkyl, alkoxy, alkylthio, or alkoxyalkyl;
Q is selected from the group consisting of a saturated or unsaturated straight or branched hydrocarbon group having a length of 1 to 9 carbon atoms, and aryl;
R₁ is selected from the group consisting of hydroxy, and 2-aminophenyl optionally substituted by one or more R₃; and
R₃ can be hydrogen, alkyl, cyano, halogen, haloalkyl, hydroxy, mercapto, alkoxy, alkylthio, alkoxyalkyl, arylalkyl, aryl or Het.

3. The thiazolidinedione-based HDAC inhibitor according to claim 1, wherein
in the Formula V, W is selected from the group consisting of an oxygen atom and a sulfur atom; X, Y, Z, M₁ and M₂ each independently represent a carbon or nitrogen atom, when X, Y, Z, M₁ or M₂ is a carbon atom, each is independently and optionally substituted by R₂, and R₂ is hydrogen, alkyl, halogen or alkoxy;
Q is selected from the group consisting of a saturated or unsaturated straight or branched hydrocarbon group having a length of 3 to 9 carbon atoms, and aryl;
R₁ is selected from the group consisting of hydroxy, and 2-aminophenyl optionally substituted by one or more R₃; and
R₃ is hydrogen, alkyl, cyano, halogen, haloalkyl, aryl or Het.

4. The thiazolidinedione-based HDAC inhibitor according to claim 1, wherein in the formula, hydrogen attached to carbon is replaced by deuterium, which is an isotope of hydrogen.

5. The thiazolidinedione-based HDAC inhibitor according to claim 2, wherein the alkyl is replaced by deuterated alkyl, the alkoxy is replaced by deuterated alkoxy, a benzene ring is replaced by a deuterated benzene ring, and an aromatic ring is replaced by a deuterated aromatic ring.

6. The thiazolidinedione-based HDAC inhibitor according to claim 1, wherein the thiazolidinedione-based HDAC inhibitor has a structure comprising:
(1) (Z)-7-(5-benzylene-2,4-dioxothiazolidin-3-yl)-N-hydroxyheptanamide;
(2) (Z)-7-(5-(2-chlorobenzylene)-2,4-dioxathiazolidin-3-yl)-N-hydroxyheptanamide;
(3) (Z)-7-(5-(3-chlorobenzylene)-2,4-dioxathiazolidin-3-yl)-N-hydroxyheptanamide;
(4) (Z)-7-(5-(4-chlorobenzylene)-2,4-dioxathiazolidin-3-yl)-N-hydroxyheptanamide;
(5) (Z)-7-(5-(2-bromobenzylene)-2,4-dioxathiazolidin-3-yl)-N-hydroxyheptanamide;
(6) (Z)-7-(5-(3-bromobenzylene)-2,4-dioxathiazolidin-3-yl)-N-hydroxyheptanamide;
(7) (Z)-7-(5-(4-bromobenzylene)-2,4-dioxathiazolidin-3-yl)-N-hydroxyheptanamide;
(8) (Z)-7-(5-(4-fluorobenzylene)-2,4-dioxathiazolidin-3-yl)-N-hydroxyheptanamide;
(9) (Z)-N-hydroxy-7-(5-(3-methoxybenzylene)-2,4-dioxathiazolidin-3-yl)heptanamide;
(10) (Z)-N-hydroxy-7-(5-(4-methoxybenzylene)-2,4-dioxathiazolidin-3-yl)heptanamide;
(11) methyl (Z)-3-((3-(7-(hydroxyamino)-7-oxoheptyl)-2,4-dioxathiazolidin-5-ylidene)methyl)benzoate;
(12) methyl (Z)-4-((3-(7-(hydroxyamino)-7-oxoheptyl)-2,4-dioxathiazolidin-5-ylidene)methyl)benzoate;
(13) (Z)-7-(5-(4-aminobenzylene)-2,4-dioxathiazolidin-3-yl)-N-hydroxyheptanamide;
(14) (Z)-7-(5-(2-ethoxybenzylene)-2,4-dioxathiazolidin-3-yl)-N-hydroxyheptanamide;
(15) (Z)-N-hydroxy-7-(5-(4-isopropylbenzylene)-2,4-dioxathiazolidin-3-yl)heptanamide;
(16) (Z)-7-(5-(4-bromo-2-methoxybenzylene)-2,4-dioxathiazolidin-3-yl)-N-hydroxyheptanamide;
(17) (Z)-7-(5-(5-bromo-2-methoxybenzylene)-2,4-dioxathiazolidin-3-yl)-N-hydroxyheptanamide;
(18) (Z)-7-(5-(3-bromo-2-methoxybenzylene)-2,4-dioxathiazolidin-3-yl)-N-hydroxyheptanamide;
(19) (Z)-7-(5-(2-bromo-4-methoxybenzylene)-2,4-dioxathiazolidin-3-yl)-N-hydroxyheptanamide;
(20) (Z)-7-(5-(3-bromo-4-methoxybenzylene)-2,4-dioxathiazolidin-3-yl)-N-hydroxyheptanamide;
(21) (Z)-7-(5-(3-chloro-2-methoxybenzylene)-2,4-dioxathiazolidin-3-yl)-N-hydroxyheptanamide;
(22) (Z)-7-(5-(4-chloro-2-methoxybenzylene)-2,4-dioxathiazolidin-3-yl)-N-hydroxyheptanamide;
(23) (Z)-7-(5-(5-chloro-2-methoxybenzylene)-2,4-dioxathiazolidin-3-yl)-N-hydroxyheptanamide;
(24) (Z)-7-(5-(6-chloro-2-methoxybenzylene)-2,4-dioxathiazolidin-3-yl)-N-hydroxyheptanamide;
(25) (Z)-N-hydroxy-7-(5-(2-methoxy-4-methylbenzylene)-2,4-dioxathiazolidin-3-yl)heptanamide;
(26) (Z)-7-(5-(4-fluoro-2-methoxybenzylene)-2,4-dioxathiazolidin-3-yl)-N-hydroxyheptanamide;
(27) (Z)-7-(5-(2,4-dimethoxybenzylene)-2,4-dioxathiazolidin-3-yl)-N-hydroxyheptanamide;
(28) (Z)-N-hydroxy-7-(5-(naphth-2-ylmethylene)-2,4-dioxathiazolidin-3-yl)heptanamide;
(29) (Z)-7-(2,4-dioxo-5-(quinol-2-ylmethylene)thiazolidin-3-yl)-N-hydroxyheptanamide;
(30) (Z)-7-(2,4-dioxo-5-(quinol-7-methylene)thiazolidin-3-yl)-N-hydroxyheptanamide;
(31) (Z)-7-(2,4-dioxo-5-(quinol-3-ylmethylene)thiazolidin-3-yl)-N-hydroxyheptanamide;
(32) (Z)-7-(2,4-dioxy-5-(quinol-6-ylmethylene)thiazolidin-3-yl)-N-hydroxyheptanamide;
(33) (Z)-7-(5-((2,3-dihydrobenzofuran-5-yl)methylene)-2,4-dioxathiazolidin-3-yl)-N-hydroxyheptanamide;
(34) (Z)-7-(5-(benzo[d][1,3]dioxazol5-ylmethylene)-2,4-dioxathiazolin-3-yl)-N-hydroxyheptanamide;
(35) (Z)-7-(2,4-dioxy-5-(pyridin-3-ylmethylene)thiazolidin-3-yl)-N-hydroxyheptanamide;
(36) (Z)-7-(2,4-dioxo-5-(quinol-8-methylene)thiazolidin-3-yl)-N-hydroxyheptanamide;
(37) (Z)-7-(2,4-dioxo-5-(thien-2-ylmethylene)thiazolidin-3-yl)-N-hydroxyheptanamide;
(38) (Z)-7-(5-(furan-2-ylmethylene)-2,4-dioxathiazolidin-3-yl)-N-hydroxyheptanamide;
(39) (Z)-N-hydroxy-7-(5-(4-methoxybenzylene)-4-oxo-2-thiothiazolidin-3-yl)heptanamide;
(40) (Z)-7-(5-(4-chlorobenzylene)-4-oxo-2-thiathiazolidin-3-yl)-N-hydroxyheptanamide;
(41) (Z)-N-hydroxy-7-(5-(4-methylbenzylene)-4-oxo-2-thiothiazolidin-3-yl)heptanamide;
(42) (Z)-7-(5-(4-ethylbenzylene)-4-oxo-2-thiothiazolidin-3-yl)-N-hydroxyheptanamide; and
(43) (Z)-N-(2-amino-4-fluorophenyl)-4-((2,4-dioxy-5-(pyridin-3-ylmethylene)thiazolidin-3-yl)methyl)benzamide.

7. A method for preparing the thiazolidinedione-based HDAC inhibitor according to claim 6, wherein a method for preparing thiazolidinedione-based HDAC inhibitors (1-15) comprises: subjecting substituted benzaldehydes (1a-1o) and thiazolidinedione, as starting materials, to a Knoevenagel condensation reaction under the action of β-alanine to obtain intermediates (2a-2o), respectively; subjecting the intermediates (2a-2o) and ethyl 7-bromoheptanoate to a nucleophilic substitution reaction to obtain key intermediates (3a-3o), respectively; hydrolyzing the intermediates (3a-3o) under acidic conditions to obtain intermediates (4a-4o), respectively; and condensing the intermediates (4a-4o) with hydroxylamine hydrochloride to obtain target compounds (1-15), respectively, with a preparation route as below:

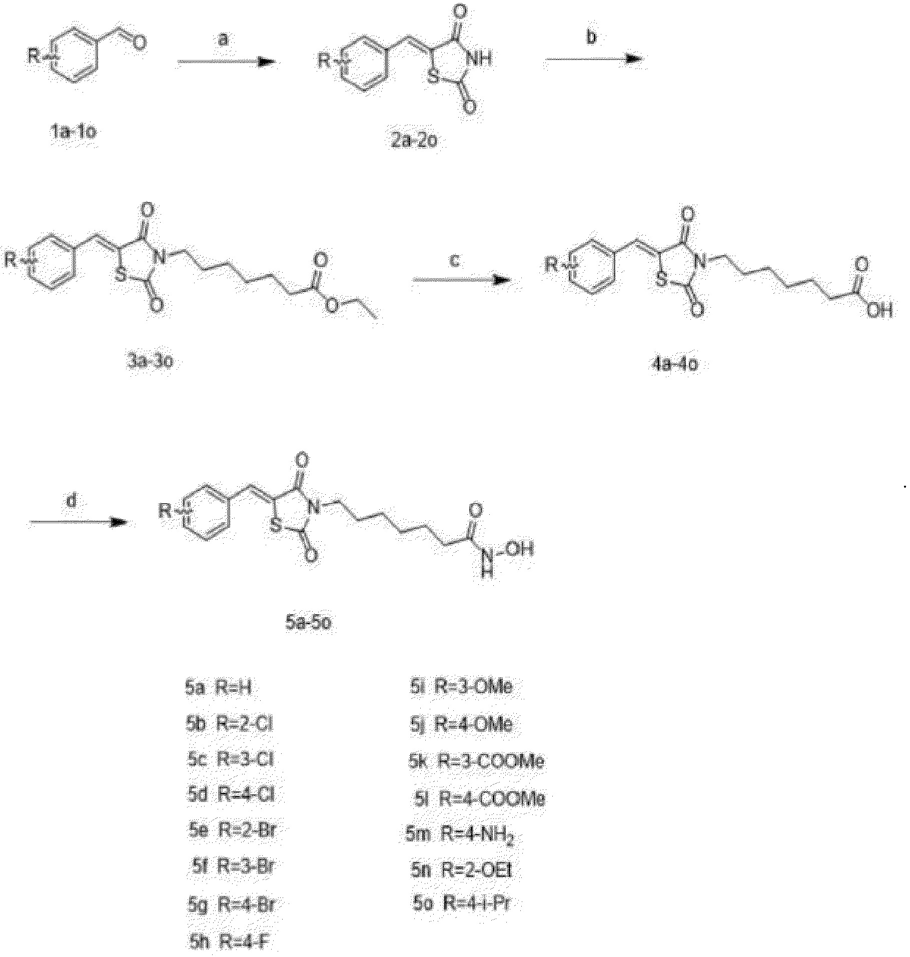

8. A method for preparing the thiazolidinedione-based HDAC inhibitor according to claim 6, wherein a method for preparing thiazolidinedione-based HDAC inhibitors (16-27) comprises: subjecting substituted benzaldehydes (6a-6I) and thiazolidinedione, as starting materials, to a Knoevenagel condensation reaction under the action of β-alanine to obtain intermediates (7a-7I), respectively; subjecting the intermediates (7a-7I) and ethyl 7-bromoheptanoate to a nucleophilic substitution reaction to obtain key intermediates (8a-8I), respectively; hydrolyzing the intermediates (8a-8I) under acidic conditions to obtain intermediates (9a-9I); and condensing the intermediates (9a-9I) with hydroxylamine hydrochloride to obtain target compounds (16-27), respectively, with a preparation route as below:

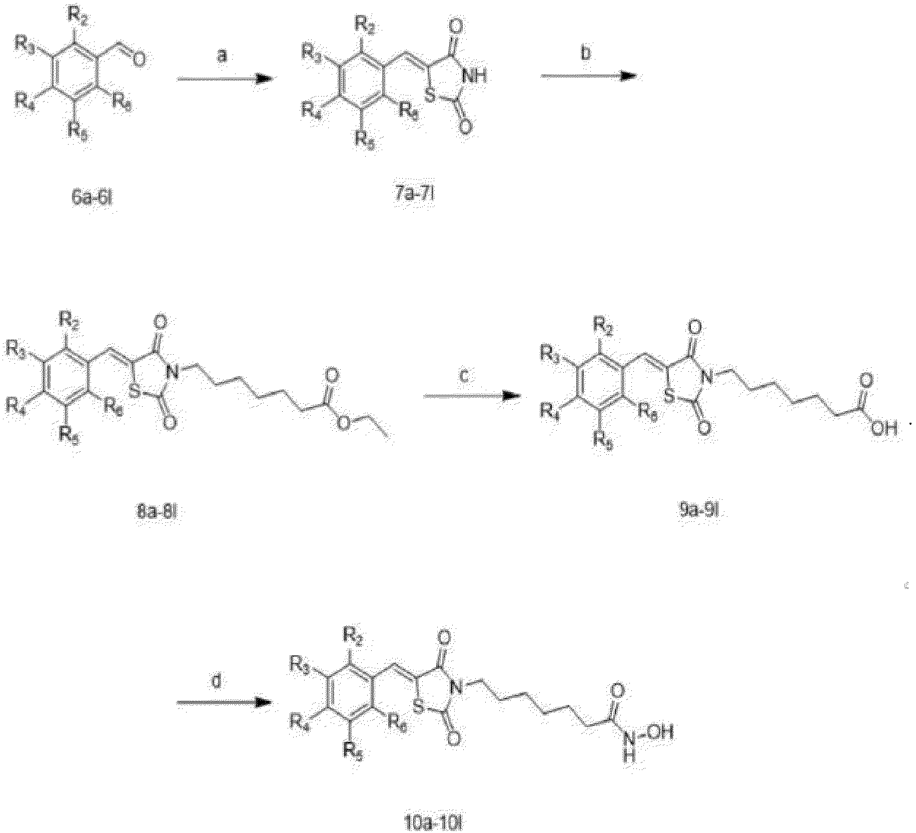

9. A method for preparing the thiazolidinedione-based HDAC inhibitor according to claim 6, wherein a method for preparing thiazolidinedione-based HDAC inhibitors (28-38) comprises: subjecting aromatic aldehyde (11a-11k) and thiazolidinedione, as starting materials, to a Knoevenagel condensation reaction under the action of β-alanine to obtain intermediates (12a-12k), respectively; subjecting the intermediates (12a-12k) and ethyl 7-bromoheptanoate to a nucleophilic substitution reaction to obtain key intermediates (13a-13k), respectively; hydrolyzing the intermediates (13a-13k) under acidic conditions to obtain intermediates (14a-14k), respectively; and condensing the intermediates (14a-14k) with hydroxylamine hydrochloride to obtain target compounds (28-38), respectively, with a preparation route as below:

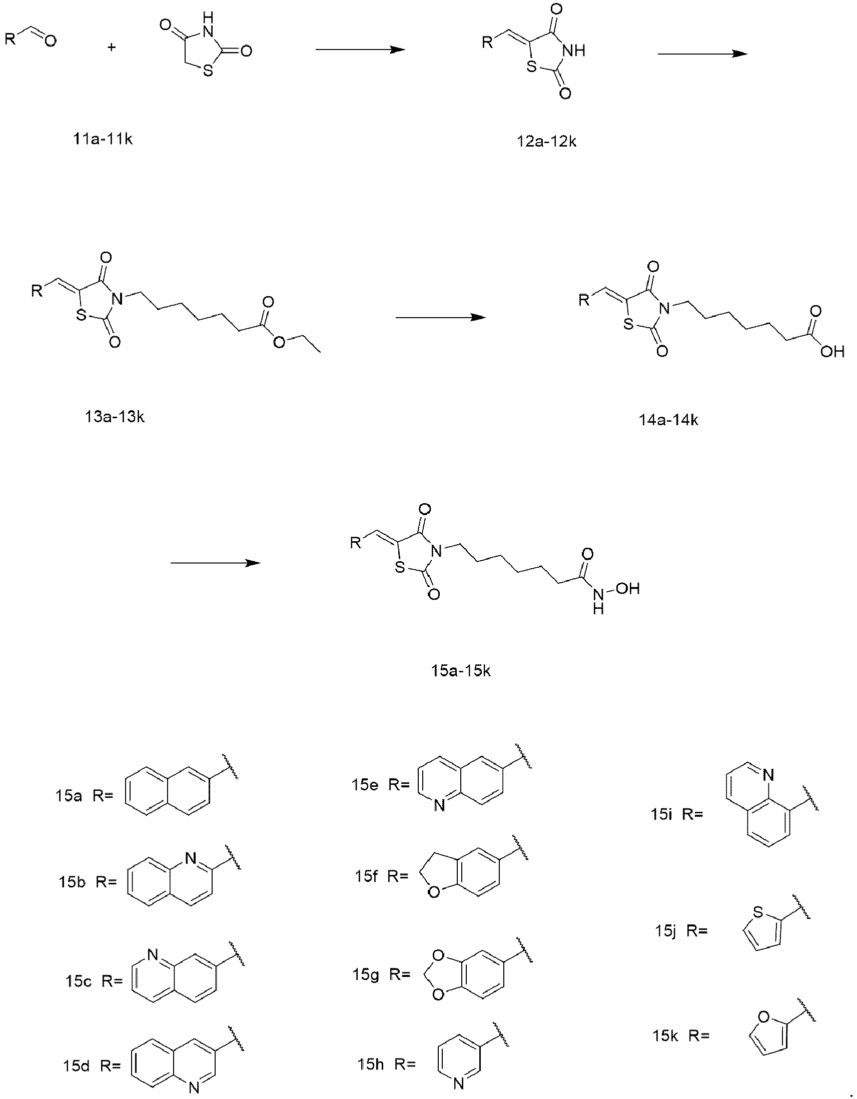

10. A method for preparing the thiazolidinedione-based HDAC inhibitor according to claim 6, wherein a method for preparing thiazolidinedione-based HDAC inhibitors (39-42) comprises: subjecting substituted benzaldehydes (16a-16d) and rhodanine, as starting materials, to a Knoevenagel condensation reaction under the action of β-alanine to obtain intermediates (17a-17d), respectively; subjecting the intermediates (17a-17d) and ethyl 7-bromoheptanoate to a nucleophilic substitution reaction to obtain key intermediates (18a-18d), respectively; hydrolyzing the intermediates (18a-18d) under acidic conditions to obtain intermediates (19a-19d), respectively; and condensing the intermediates (19a-19d) with hydroxylamine hydrochloride to obtain target compounds (39-42), respectively, with a preparation route as below:

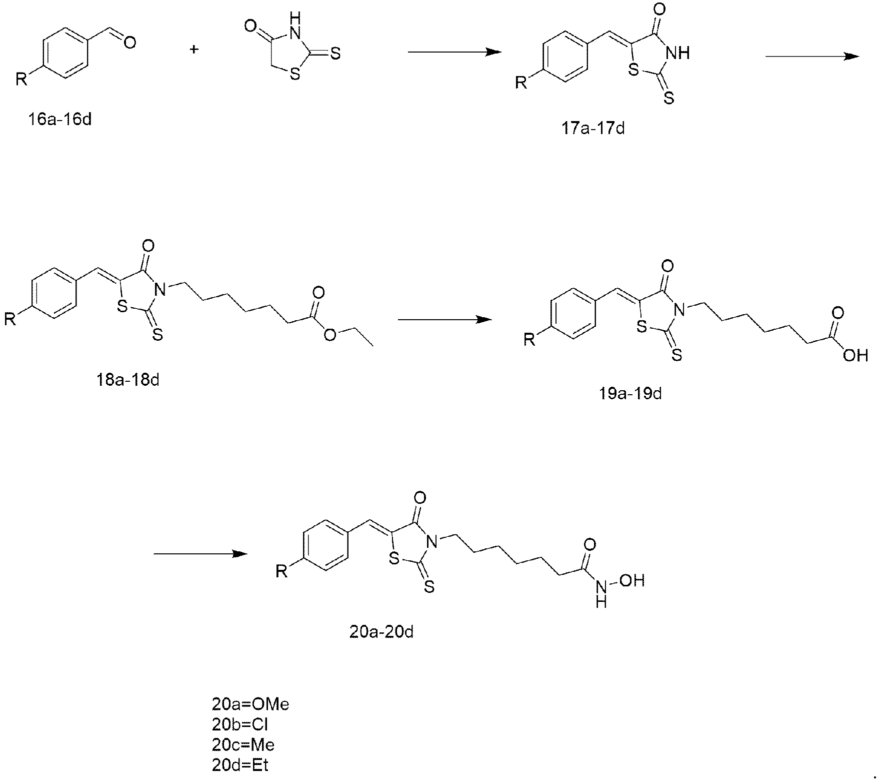

11. A pharmaceutical composition, comprising the compound according to any one of claims 1 to 6 or a pharmaceutically acceptable salt thereof, and a pharmaceutically acceptable carrier, wherein the compound according to any one of claims 1 to 6 or the pharmaceutically acceptable salt thereof is an active ingredient.

12. Use of the compound according to any one of claims 1 to 6 in the preparation of a medicament for preventing or treating HDAC-associated clinical disorders.

13. The use of the compound according to claim 12, wherein the HDAC-associated clinical disorders comprise lung cancer, melanoma, liver cancer, kidney cancer, leukemia, prostate cancer, thyroid cancer, skin diseases, pancreatic cancer, ovarian cancer, testicular cancer, breast cancer, bladder cancer, gallbladder cancer, myelodysplastic syndrome, lymphoma, esophageal cancer, gastrointestinal cancer, astrocytoma, neuroblastoma, glioma, neurilemoma, mesothelioma, non-insulin-dependent diabetes mellitus, and autoimmune diseases.
